# EUROPEAN PATENT APPLICATION

(11) **EP 0 781 777 A1**
(43) Date of publication of application: **02.07.1997**
(21) Application number: 96120682.8
(22) Date of filing: 20.12.1996
(51) Int. Cl.: C07F 7/08, C08F 230/08, G02B 1/04, A61L 27/00

(54) **Silicon-containing compound and ocular lens material**

(30) Priority: 28.12.1995 JP 343604/95; 09.01.1996 JP 1645/96
(71) Applicant: Menicon Co., Ltd., Nagoya-shi Aichi-ken (JP)
(72) Inventor: Yokoyama, Yasuhiro, Menicon Co., Ltd., Kasugai-shi, Aichi-ken (JP); Watanabe, Yuriko, Menicon Co., Ltd., Kasugai-shi, Aichi-ken (JP)
(74) Representative: Wächtershäuser, Günter, Prof. Dr.

(57) **Abstract**

A silicon-containing compound of the formula (I): 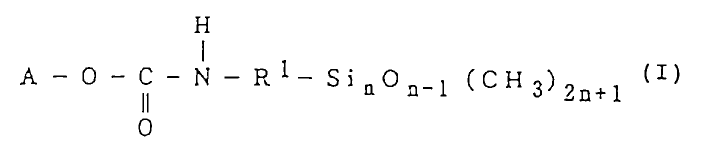 wherein R¹ is a C₁₋₆ alkylene group; A is a group of the formula (II): 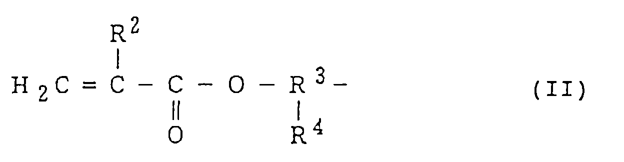 wherein R² is a hydrogen atom or a methyl group, R³ is a C₁₋₆ trivalent hydrocarbon group or a C₁₋₆ bivalent hydrocarbon group when R⁴ is a hydrogen atom, and R⁴ is a hydrogen atom, a hydroxyl group, a C₁₋₆ alkyl group or a group of the formula (III): 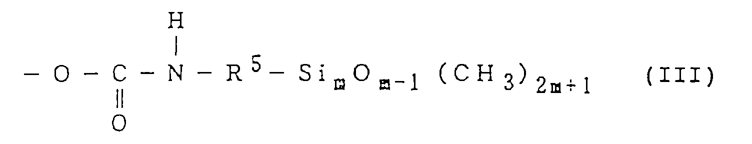 wherein R⁵ is a C₁₋₆ alkylene group, and m is an integer of from 1 to 15, or a group of the formula (IV): 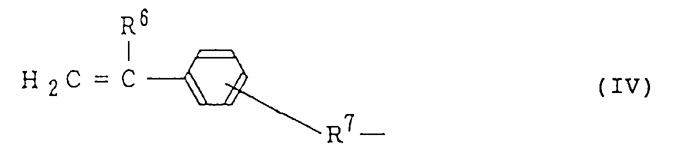 wherein R⁶ is a hydrogen atom or a methyl group, and R⁷ is a direct bond or a C₁₋₆ alkylene group; and n is an integer of from 1 to 15.

## Description

The present invention relates to a silicon-containing compound and an ocular lens material. More particularly, it relates to a silicon-containing compound useful for a silicon-containing polymer which is excellent not only in oxygen permeability, transparency and surface hydrophilic property but also in release property, dimensional stability, durability, sliding property, low friction property, anti-thrombogenic property, etc. and which is useful not only for a fiber-treating agent, a coating material, a silicon-modified rubber material or a surface-treating agent but also for a highly oxygen permeable molded product, particularly an ocular lens material such as a contact lens material, an intraocular lens material or an artificial cornea material, and an ocular lens material which not only is excellent in oxygen permeability and transparency and simultaneously has a hydrophilic property, but also is excellent in release property, dimensional stability, durability, sliding property, low friction property and anti-thrombogenic property and which is useful for a contact lens, an intraocular lens or an artificial cornea.

Heretofore, it has been known that an acrylic polymer having polysiloxane groups introduced, has excellent light resistance, release property and water repellency. Accordingly, various molded products have been proposed wherein such properties of an acrylic polymer having polysiloxane groups introduced, are utilized. Among them, a transparent material for optical applications, such as an ocular lens material, is being developed.

As such an ocular lens material, a material composed essentially of polymethyl methacrylate has, for example, been practically used. However, such a material composed essentially of polymethyl methacrylate has a problem that it is inferior in oxygen permeability, although it has substantial merits in that it is excellent in mechanical properties and optical properties such as transparency.

A contact lens made of such an ocular lens material which is inferior in oxygen permeability, reduces the amount of oxygen supplied to the cornea. Accordingly, if such a contact lens is put on the eye over a long period of time, hyperemia, edema or other corneal troubles are likely to be caused.

In order to improve the oxygen permeability of a methacrylate type polymer such as the above-mentioned polymethyl methacrylate, it has been proposed to employ a monomer having a large amount of silicon atoms introduced into the molecule of the methacrylate monomer. For example, a contact lens has been proposed which is made of a copolymer of a methacrylate monomer with an oligosiloxanyl alkyl (meth)acrylate type monomer having an oligosiloxane bond introduced into the molecule of the methacrylate type monomer.

However, such a contact lens still does not satisfy the oxygen permeability required for an ocular lens such as a contact lens, although its oxygen permeability is improved over a conventional contact lens made of a methacrylate type polymer such as polymethyl methacrylate. Accordingly, it has been still desired to develop a material having excellent oxygen permeability.

Further, when it is attempted to improve the oxygen permeability of a material, there is usually an additional problem such that the hydrophilic property such as wettability is likely to decrease so much that the material will not have a sufficient hydrophilic property as required for an ocular lens material.

Particularly, the above-mentioned polymer obtained by using the oligosiloxanylalkyl (meth)acrylate type monomer as an essential component, is inferior in wettability. Accordingly, in order to obtain an ocular lens from such a polymer, it is necessary to carry out a cumbersome secondary surface modification, for example, by discharge treatment such as plasma treatment.

The present invention has been made in view of the above-described prior art, and it is an object of the present invention to provide a novel compound which is useful for a polymer excellent in oxygen permeability, transparency, surface hydrophilic property such as wettability, release property, dimensional stability and durability simultaneously and thus useful for e.g. highly oxygen permeable molded products such as ocular lens materials and which is excellent also in the polymerizability. Another object of the present invention is to provide an ocular lens material which is particularly excellent in oxygen permeability and transparency and simultaneously has a proper hydrophilic property such as wettability and which is simultaneously excellent in release property, dimensional stability and durability.

The present invention provides a silicon-containing compound of the formula (I): 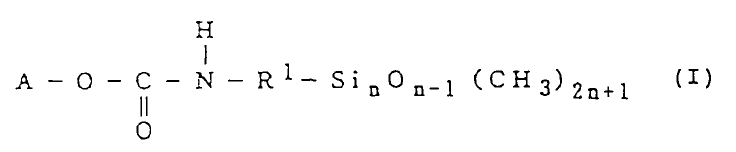 wherein R¹ is a C₁₋₆ alkylene group; A is a group of the formula (II): 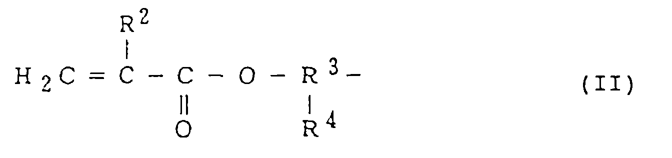 wherein R² is a hydrogen atom or a methyl group, R³ is a C₁₋₆ trivalent hydrocarbon group or a C₁₋₆ bivalent hydrocarbon group when R⁴ is a hydrogen atom, and R⁴ is a hydrogen atom, a hydroxyl group, a C₁₋₆ alkyl group or a group of the formula (III): 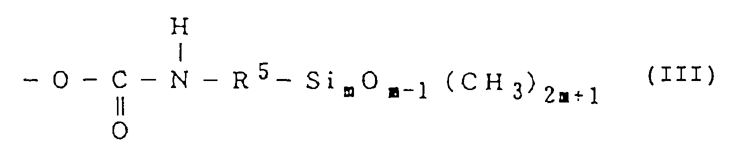 wherein R⁵ is a C₁₋₆ alkylene group, and m is an integer of from 1 to 15, or a group of the formula (IV): 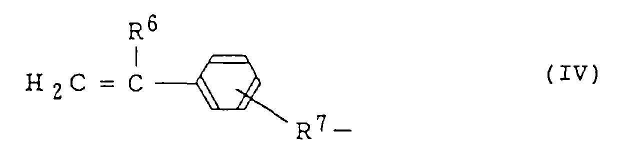 wherein R⁶ is a hydrogen atom or a methyl group, and R⁷ is a direct bond or a C₁₋₆ alkylene group; and n is an integer of from 1 to 15.

Further, the present invention provides an intraocular lens material made of a polymer obtained by polymerizing polymerizable components containing such a silicon-containing compound.

In the accompanying drawings:
Figure 1 is a chart showing the transmittance of infrared rays by N-tris(trimethylsiloxy)silylpropyl methacryloxyethyl carbamate obtained in Example 1.
Figure 2 shows the ¹H-NMR spectrum of N-tris(trimethylsiloxy)silylpropylmethacryloxyethyl carbamate obtained in Example 1.
Figure 3 shows the mass spectrum of N-tris(trimethylsiloxy)silylpropylmethacryloxyethyl carbamate obtained in Example 1.
Figure 4 is a chart showing the transmittance of infrared rays by N-tris(trimethylsiloxy)silylpropyl acryloxyethyl carbamate obtained in Example 2.
Figure 5 shows the ¹H-NMR spectrum of N-tris(trimethylsiloxy)silylpropylacryloxyethyl carbamate obtained in Example 2.
Figure 6 shows the mass spectrum of N-tris(trimethylsiloxy)silylpropylacryloxyethyl carbamate obtained in Example 2.

Now, the present invention will be described in detail with reference to the preferred embodiments.

As mentioned above, the silicon-containing compound of the present invention is a silicon-containing compound of the formula (I): 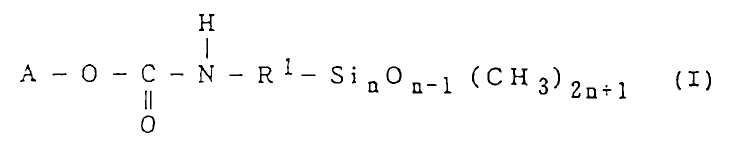 wherein R¹ is a C₁₋₆ alkylene group; A is a group of the formula (II): 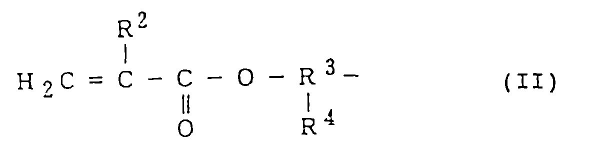 wherein R² is a hydrogen atom or a methyl group, R³ is a C₁₋₆ trivalent hydrocarbon group or a C₁₋₆ bivalent hydrocarbon group when R⁴ is a hydrogen atom, and R⁴ is a hydrogen atom, a hydroxyl group, a C₁₋₆ alkyl group or a group of the formula (III): 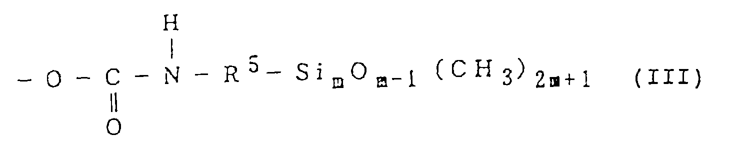 wherein R⁵ is a C₁₋₆ alkylene group, and m is an integer of from 1 to 15, or a group of the formula (IV): 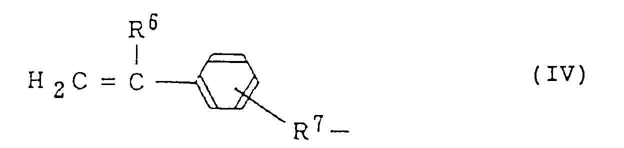 wherein R⁶ is a hydrogen atom or a methyl group, and R⁷ is a direct bond or a C₁₋₆ alkylene group; and n is an integer of from 1 to 15, and the ocular lens material of the present invention is the one made of a polymer obtained by polymerizing polymerizable components containing such a silicon-containing compound.

The silicon-containing compound of the formula (I) has two polar groups in its molecule and thus has not only homopolymerizability but also copolymerizability. Further, it has good compatibility with both a hydrophobic component and a hydrophilic component. It is a compound which imparts excellent oxygen permeability and transparency as well as a proper hydrophilic property to the resulting polymer or ocular lens material and which also has a nature of imparting release property, dimensional stability and durability to such a polymer or ocular lens material.

In the formula (I), R¹ is a C₁₋₆ alkylene group. However, it is preferably a C₂₋₄ alkylene group from the viewpoint of the compatibility of the silicon-containing compound with other polymerizable components or good processability of the resulting polymer. If the alkylene group for R¹ has a carbon number of 7 or more, the viscosity of the silicon-containing compound tends to increase too much, whereby the handling tends to be difficult, and the resulting polymer tends to be too soft, whereby the processability tends to be poor.

In the formula (I), A is a group of the formula (II) or a group of the formula (IV). In the formula (II), R³ is a C₁₋₆ trivalent hydrocarbon group or a C₁₋₆ bivalent hydrocarbon group when R⁴ is a hydrogen atom. However, it is preferably a C₂₋₄ trivalent hydrocarbon group or a C₂₋₄ bivalent hydrocarbon group in view of the compatibility of the silicon-containing compound with other polymerizable components or good processability of the resulting polymer. If the trivalent hydrocarbon group or the bivalent hydrocarbon group for R³ has a carbon number of 7 or more, the viscosity of the silicon-containing compound tends to increase too much, whereby the handling tends to be difficult, and the resulting polymer tends to be too soft, whereby the processability tends to be poor.

In the formula (II), R⁴ is usually a hydrogen atom, but it may be a hydroxyl group or a group of the formula (III) (hereinafter referred to as a SiU group) from such a viewpoint that the effect for imparting wettability is thereby large. In the SiU group, R⁵ is a C₁₋₆ alkylene group, but it is preferably a C₂₋₄ alkylene group in view of the compatibility of the silicon-containing compound with other polymerizable components or good processability of the resulting polymer. If the alkylene group for R⁵ has a carbon number of 7 or more, the viscosity of the silicon-containing compound tends to increase too much, whereby the handling tends to be difficult, and the resulting polymer tends to be too soft, whereby the processability tends to be poor. In the SiU group, m is an integer of from 1 to 15, but it is preferably an integer of from 2 to 10, more preferably an integer of from 3 to 6, in view of the compatibility of the silicon-containing compound with other polymerizable components or good processability of the resulting polymer. If m is an integer of 16 or higher, the viscosity of the silicon-containing compound tends to increase too much, whereby the handling tends to be difficult, and the resulting polymer tends to be too soft, whereby the processability tends to be poor.

In the formula (I), n is an integer of from 1 to 15. However, it is preferably an integer of from 2 to 10, more preferably an integer of from 3 to 6, in view of the compatibility of the silicon-containing compound with other polymerizable components or good processability of the resulting polymer. If n is an integer of 16 or higher, the viscosity of the silicon-containing compound tends to increase too much, whereby the handling tends to be difficult, and the resulting polymer tends to be too soft, whereby the processability tends to be poor.

Specific examples of the above silicon-containing compound include N-pentamethyldisiloxanylmethyl (meth)acryloxyethyl carbamate, N-trimethylsiloxydimethylsilylpropyl (meth)acryloxyethyl carbamate, N-methylbis(trimethylsiloxy)silylpropyl (meth)acryloxyethyl carbamate, N-tris(trimethylsiloxy)silylpropyl (meth)acryloxyethyl carbamate, N-mono[methylbis(trimethylsiloxy)siloxy]bis (trimethylsiloxy)silylpropyl (meth)acryloxyethyl carbamate, N-tris[methylbis(trimethylsiloxy)siloxy] silylpropyl (meth)acryloxyethyl carbamate, N-trimethylsilylmethyl (meth)acryloxyethyl carbamate, N-trimethylsilylpropyl (meth)acryloxyethyl carbamate, N-methylbis(trimethylsiloxy)silylethyltetramethyl disiloxanylmethyl (meth)acryloxyethyl carbamate, N-trimethylsiloxydimethylsilylpropyl (meth)acryloxyethyl carbamate, N-pentamethyldisiloxanylmethylvinylphenyl carbamate, N-trimethylsiloxydimethylsilylpropylvinyl phenyl carbamate, N-methylbis(trimethylsiloxy)silylpropyl vinylphenyl carbamate, N-tris(trimethylsiloxy)silylpropyl vinylphenyl carbamate, N-mono[methylbis(trimethylsiloxy) siloxy]bis(trimethylsiloxy)silylpropylvinylphenyl carbamate, N-tris[methylbis(trimethylsiloxy)siloxy]silyl propylvinylphenyl carbamate, N-trimethylsilylmethylvinyl phenyl carbamate, N-trimethylsilylpropylvinylphenyl carbamate, N-methylbis(trimethylsiloxy)silylethyl tetramethyldisiloxanylmethylvinylphenyl carbamate and N-trimethylsiloxydimethylsilylpropylvinylphenyl carbamate. These compounds may be used alone or in combination as a mixture of two or more of them.

In this specification, "(meth)acryl..." means "acryl... and/or methacryl...".

The excellent polymerizability of the above silicon-containing compound is attributable to the fact that it contains a polymerizable unsaturated double bond such as an acryloyl group, a methacryloyl group or a vinylphenyl group, in its structure.

Further, the silicon-containing compound contains silicon in its structure, and a polymer obtained by polymerizing such a compound has excellent oxygen permeability.

Furthermore, the silicon-containing compound not only has an urethane bond but also contains a hydroxyl group as R⁴ in the formula (II) or a urethane bond in the SiU group as R⁴, in its structure. A polymer obtainable by polymerizing such a compound also shows an excellent hydrophilic property.

The silicon-containing compound of the present invention can be prepared, for example, by a method of reacting a hydroxyl group-containing monomer with a silicon-containing isocyanate compound by means of a suitable catalyst for the reaction.

Typical examples of such a hydroxyl group-containing monomer include a hydroxyl group-containing styrene monomer such as p-hydroxystyrene; and a hydroxyl group-containing (meth)acrylate such as 2,3-dihydroxypropyl (meth)acrylate, 2-hydroxyethyl (meth)acrylate, 2-hydroxypropyl (meth)acrylate, 2-hydroxybutyl (meth)acrylate or hydroxyoctyl (meth)acrylate.

Typical examples of the above silicon-containing isocyanate compound include compounds of the formula (V):

O=C=N-R⁸-SiᵣOᵣ₋₁(CH₃)2ᵣ₊₁ (V)

wherein R⁸ is a C₁₋₆ linear or branched alkylene group, and r is an integer of from 1 to 15, such as pentamethyldisiloxanylmethyl isocyanate, trimethylsiloxydimethylsilylpropyl isocyanate, methylbis(trimethylsiloxy)silylpropyl isocyanate, tris(trimethylsiloxy)silylpropyl isocyanate, mono[methylbis(trimethylsiloxy)siloxy]bis(trimethyl siloxy)silylpropyl isocyanate, tris[methylbis(trimethylsiloxy)siloxy]silylpropyl isocyanate, trimethylsilylmethyl isocyanate, trimethylsilylpropyl isocyanate, methylbis(trimethylsiloxy)silylethyltetramethyl disiloxanylmethyl isocyanate, tetramethyltriisopropylcyclotetrasiloxanylpropyl isocyanate, tetramethyltriisopropylcyclotetrasiloxybis (trimethylsiloxy)silylpropyl isocyanate and trimethylsiloxydimethylsilylpropyl isocyanate. As the catalyst for the reaction, a metal type catalyst such as dibutyltin dilaurate or Iron (III) Acetylacetonate, or an amine type catalyst such as triethylamine, may, for example, be preferably used. When the above hydroxyl group-containing monomer is reacted with the silicon-containing isocyanate compound, the types and blend proportions of the two compounds, the conditions such as the reaction temperature and reaction time, the type of the catalyst to be used, etc., may suitably be adjusted depending upon the type of the desired silicon-containing compound.

For example, taking into consideration the fact that an excessive amount of the hydroxyl group-containing monomer can readily be removed by washing with water at the time of purifying the reaction product, it is preferred to use the hydroxyl group-containing monomer in an amount of from 1 to 4 mols per mol of the silicon-containing isocyanate compound and to carry out the reaction in such a manner that the above-mentioned catalyst for the reaction is charged to the hydroxyl group-containing monomer, then the silicon-containing isocyanate compound is dropwise added thereto while maintaining the system at a temperature of from 20 to 50°C, and stirring is continued for from 10 to 120 hours at a temperature of from 20 to 50°C.

The temperature for the reaction is more preferably from 30 to 40°C to let the reaction proceed sufficiently and to avoid a danger of letting the reaction proceed too vigorously.

Further, if water is present in the reaction system during such a reaction, the reaction sometimes tends to hardly proceed. Accordingly, it is preferred to maintain the reaction system in a dried state.

After conducting the reaction as described above, if an unreacted material is present, such a material is removed by washing with water, as mentioned above, and the product is then dried to obtain the desired silicon-containing compound.

When the silicon-containing compound of the present invention thus obtained, is used as a polymerizable component, it is possible to obtain a polymer which is simultaneously excellent in oxygen permeability, transparency and surface hydrophilic property and which is excellent also in release property, dimensional stability, durability, sliding property, low friction property, anti-thrombogenic property, etc.

To obtain such a polymer from the silicon-containing compound of the present invention, polymerizable components comprising the silicon-containing compound and proper amounts of optional other monomers copolymerizable with the silicon-containing compound, a crosslinking agent, etc., as the case requires, may be polymerized by usual bulk polymerization or solution polymerization by means of e.g. a radical polymerization initiator or a photopolymerization initiator.

The polymer thus obtained from the silicon-containing compound of the present invention is the one having excellent physical properties imparted as mentioned above. Accordingly, such a polymer is useful, for example, not only for a fiber-treating agent, a coating material, a silicone-modified rubber material or a surface-treating agent but also for a highly oxygen permeable molded product, particularly an ocular lens material such as a contact lens material, an intraocular lens material or an artificial cornea material.

The content of the above silicon-containing compound is preferably adjusted to be at least 5 wt%, preferably at least 10 wt%, more preferably at least 20 wt%, of the total amount of the polymerizable components to obtain sufficient effects for imparting excellent oxygen permeability and a proper hydrophilic property to the ocular lens material. In the present invention, the total amount of the polymerizable components may be constituted by the silicon-containing compound. However, in view of the mechanical strength and processability of the resulting polymer, the content of the silicon-containing compound is preferably adjusted to be at most 95 wt%, preferably at most 90 wt%, of the total amount of the polymerizable components.

As mentioned above, the ocular lens material of the present invention may be a polymer obtained by polymerizing polymerizable components comprising the silicon-containing compound of the formula (I) only. However, it may be the one made of a polymer obtained by polymerizing polymerizable components which further contain a polymerizable monomer having an unsaturated double bond copolymerizable with the silicon-containing compound (hereinafter referred to as the polymerizable monomer). The type of the polymerizable monomer is not particularly limited, so long as it does not impair the purpose of the present invention.

The polymerizable monomer may be selected suitably depending upon the nature of the desired ocular lens material, and its content may be adjusted suitably so that the total amount of polymerizable components will be 100 wt%. For example, to obtain a water non-absorptive ocular lens material, a hydrophobic monomer or macromonomer may be selected for use, and to obtain a water-absorptive ocular lens material, a hydrophilic monomer or macromonomer may be selected for use. Further, to obtain an ocular lens material excellent in mechanical strength, a reinforcing monomer or macromonomer may be selected for use, and to obtain an ocular lens material excellent in water resistance or solvent resistance, a crosslinkable monomer for forming a crosslinked structure may be selected for use. Specifically, for example, in a case where it is desired to further impart oxygen permeability to the resulting ocular lens material and at the same time to reinforce the mechanical strength of the ocular lens material, a polysiloxane macromonomer, such as a polysiloxane macromonomer having a polymerizable group bonded to the siloxane main chain via one or two urethane bonds, a polysiloxane macromonomer having a polymerizable group bonded directly to the siloxane main chain or a polysiloxane macromonomer having a polymerizable group bonded to the siloxane main chain via an alkylene group, may be used.

Such a polysiloxane macromonomer may, for example, be a macromonomer of the formula (V):

A¹-(U²)m₂-S³-(T⁴)ₘ₄-(U⁵)ₘ₅-A⁶ (V)

wherein A¹ is a group of the formula:

Y¹¹-R¹²-

wherein Y¹¹ is an acryloyloxy group, a methacryloyloxy group, a vinyl group or an allyl group, and R¹² is a C₁₋₆ linear or branched alkylene group;
A⁶ is a group of the formula:

-R⁶²-Y⁶¹

wherein R⁶² is a C₁₋₆ linear or branched alkylene group, and Y⁶¹ is an acryloyloxy group, a methacryloyloxy group, a vinyl group or an allyl group;
U² is a group of the formula:

-X²¹-E²²-X²³-R²⁴-

wherein X²¹ is a covalent bond, an oxygen atom or a C₁₋₆ alkylene glycol group, E²² is a -NHCO- group or a bivalent group wherein the terminals derived from a diisocyanate selected from a saturated aliphatic type, an alicyclic type and an aromatic type, are a -CONH- group and a -NHCO- group, X²³ is an oxygen atom, a C₁₋₆ alkylene glycol group or a group of the formula: 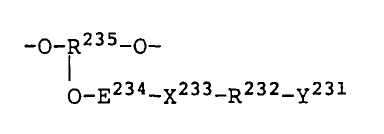 wherein R²³⁵ is a C₁₋₆ trivalent hydrocarbon group, R²³² is a C₁₋₆ linear or branched alkylene group, E²³⁴ is a -CONH- group or a bivalent group wherein the terminals derived from a diisocyanate selected from a saturated aliphatic type, an alicyclic type and an aromatic type, are a -CONH- group and a -NHCO- group, X²³³ is a covalent bond, an oxygen atom or a C₁₋₆ alkylene glycol group, and Y²³¹ is an acryloyloxy group, a methacryloyloxy group, a vinyl group or an allyl group, provided that X²³³ is a covalent bond when the adjacent E²³⁴ is a -CONH- group, or an oxygen atom or a C₁₋₆ alkylene glycol group when the adjacent E²³⁴ is a bivalent group derived from the isocyanate, and E²³⁴ forms a urethane bond between the adjacent oxygen atom and X²³³, and R²⁴ is a C₁₋₆ linear or branched alkylene group, provided that X²¹ is a covalent bond when the adjacent E²² is a -NHCO- group, or an oxygen atom or a C₁₋₆ alkylene glycol group when the adjacent E²² is the bivalent group derived from the diisocyanate, and E²² forms an urethane bond between the adjacent E²¹ and X²³; U⁵ is a group of the formula:

-R⁵⁴-X⁵³-E⁵²⁻X⁵¹-

wherein R⁵⁴ is a C₁₋₆ linear or branched alkylene group, X⁵³ is an oxygen atom, a C₁₋₆ alkylene glycol group or a group of the formula: 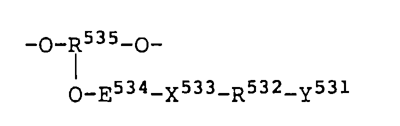 wherein R⁵³⁵ is a C₁₋₆ trivalent hydrocarbon group, R⁵³² is a C₁₋₆ linear or branched alkylene group, E⁵³⁴ is a -CONH- group or a bivalent group wherein the terminals derived from a diisocyanate selected from a saturated aliphatic type, an alicyclic type and an aromatic type, are a -CONH- group and a -NHCO- group, X⁵³³ is a covalent bond, an oxygen atom or a C₁₋₆ alkylene glycol group, Y⁵³¹ is an acryloyloxy group, a methacryloyloxy group, a vinyl group or an allyl group, provided that X⁵³³ is a covalent bond when the adjacent E⁵³⁴ is a -CONH- group, or an oxygen atom or a C₁₋₆ alkylene glycol group when the adjacent E⁵³⁴ is a bivalent group derived from the the diisocyanate, and E⁵³⁴ forms an urethane bond between the adjacent oxygen atom and X⁵³³, E⁵² is a -CONH- group or a bivalent group wherein the terminals derived from a diisocyanate selected from an unsaturated aliphatic type, an alicyclic type and an aromatic type, are a -CONH-group and a -NHCO- group, and X⁵¹ is a covalent bond, an oxygen atom or a C₁₋₆ alkylene glycol group, provided that X⁵¹ is a covalent bond when the adjacent E⁵² is a -CONH- group, or an oxygen atom or a C₁₋₆ alkylene glycol group when the adjacent E⁵² is a bivalent group derived from the diisocyanate, and E⁵² forms a urethane bonds between the adjacent X⁵¹ and E⁵³;
S³ is a group of the formula: 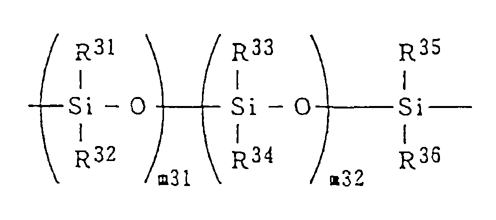 wherein each of R³¹, R³², R³³, R³⁵ and R³⁶ which are independent of one another, is a C₁₋₆ linear or branched alkyl group, of which some or all of hydrogen atoms may be substituted by fluorine atoms, or a phenyl group, R³⁴ is a C₁₋₆ linear or branched alkyl group, of which some or all of hydrogen atoms may be substituted by fluorine atoms, a phenyl group or a group of the formula: 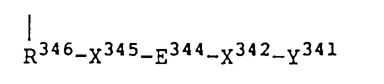 wherein each of R³⁴⁶ and R³⁴² which are independent of each other, is a C₁₋₆ linear or branched alkylene group, each of X³⁴⁵ and X³⁴³ is a covalent bond, an oxygen atom or a C₁₋₆ alkylene glycol group, E³⁴⁴ is a -CONH- group or a bivalent group wherein the terminals derived from a diisocyanate selected from a saturated aliphatic type, an alicyclic type and an aromatic type, are a -CONH- group and a -NHCO- group, and Y³⁴¹ is an acryloyloxy group, a methacryloyloxy group, a vinyl group or an allyl group, provided that each of X³⁴⁵ and X³⁴³ is a covalent bond when the adjacent E³⁴⁴ is a -CONH- group, or an oxygen atom or a C₁₋₆ alkylene glycol group when the adjacent E³⁴⁴ is a bivalent group derived from the diisocyanate, and E³⁴⁴ forms an urethane bond between the adjacent X³⁴⁵ and X³⁴³ provided that a case where all of R³¹, R³², R³³, R³⁴, R³⁵ and R³⁶ are simultaneously C₁₋₆ linear or branched alkyl groups wherein some or all of hydrogen atoms are substituted by fluorine atoms, or phenyl groups, is excluded, m31 is an integer of from 1 to 100, m32 is an integer of from 0 to (100-(m31)), provided that (m31)+(m32) is an integer of from 1 to 100; and
T⁴ is a group of the formula:

-U⁴¹-S⁴²-

wherein U⁴¹ is a group of the formula:

-R⁴¹¹-X⁴¹²-E⁴¹³-X⁴¹⁴-R⁴¹⁵-

wherein each of R⁴¹¹ and R⁴¹⁵ which are independent of each other, is a C₁₋₆ linear or branched alkylene group, each of X⁴¹² and X⁴¹⁴ which are independent of each other, is an oxygen atom or a C₁₋₆ alkylene glycol group, and E⁴¹³ is a bivalent group wherein the terminals derived from a diisocyanate selected from a saturated aliphatic type, an alicyclic type and an aromatic type, are a -CONH- group and a -NHCO- group, provided that E⁴¹³ forms an urethane bond between the adjacent X⁴¹² and X⁴¹⁴, and S⁴² is a group of the formula: 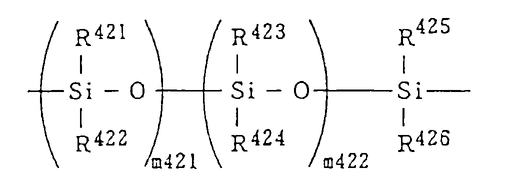 wherein each of R⁴²¹, R^{422,} R⁴²³, R⁴²⁵ and R⁴²⁶ which are independent of one another, is a C₁₋₆ linear or branched alkyl group wherein some or all of hydrogen atoms may be substituted by fluorine atoms, or a phenyl group, R⁴²⁴ is a C₁₋₆ linear or branched alkyl group wherein some or all of hydrogen atoms may be substituted by fluorine atoms, a phenyl group or a group of the formula: 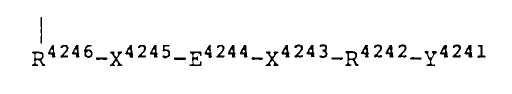 wherein each of R⁴²⁴⁶ and R⁴²⁴² which are independent of each other, is a C₁₋₆ linear or branched alkylene group, each of X⁴²⁴⁵ and X⁴²⁴³ is a covalent bond, an oxygen atom or a C₁₋₆ alkylene glycol group, E⁴²⁴⁴ is a -CONH- group or a bivalent group wherein the terminals derived from a diisocyanate selected from a saturated aliphatic type, an alicyclic type and an aromatic type, are a -CONH- group and a -NHCO- group, and Y⁴²⁴¹ is an acryloyloxy group, a methacryloyloxy group, a vinyl group or an allyl group, provided that each of X⁴²⁴⁵ and X⁴²⁴³ is a covalent bond when the adjacent E⁴²⁴⁴ is a -CONH- group, or an oxygen atom or a C₁₋₆ alkylene glycol group when the adjacent E⁴²⁴⁴ is a bivalent group derived from the diisocyanate, and E⁴²⁴⁴ forms an urethane bond between the adjacent X⁴²⁴⁵ and X⁴²⁴³, provided that a case wherein all of R⁴²¹, R⁴²², R⁴²³, R⁴²⁴, R⁴²⁵ and R⁴²⁶ are simultaneously C₁₋₆ linear or branched alkyl groups wherein some or all of hydrogen atoms are substituted by fluorine atoms, or phenyl groups, is excluded, m421 is an integer of from 1 to 100, and m422 is an integer of from 0 to (100-(m421), provided that (m421)+(m422) is an integer of from 1 to 100, or a group of the formula: 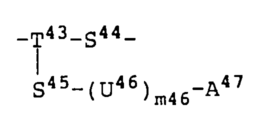 wherein T⁴³ is a group of the formula: 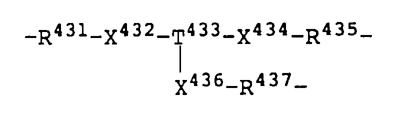 wherein each of R⁴³¹, R⁴³⁵ and R⁴³⁷ which are independent of one another, is a C₁₋₆ linear or branched alkylene group, each of X⁴³², X⁴³⁴ and X⁴³⁶ which are independent of one another, is an oxygen atom or a C₁₋₆ alkylene glycol group, and T⁴³³ is a group of the formula: 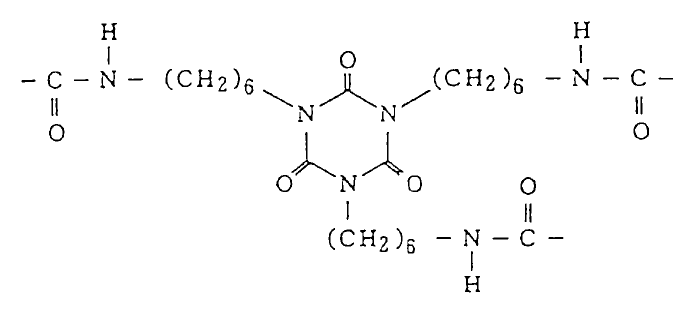 provided that it forms a urethane bond among the adjacent X⁴³², X⁴³⁴ and X⁴³⁶, S⁴⁴ is a group of the formula: 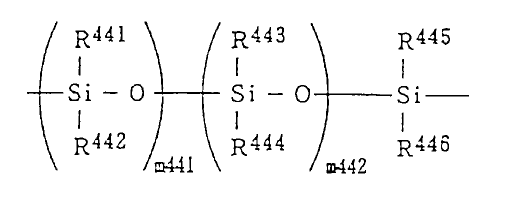 wherein each of R⁴⁴¹, R⁴⁴², R⁴⁴³, R⁴⁴⁵ and R⁴⁴⁶ which are independent of one another is a C₁₋₆ linear or branched alkyl group wherein some or all of hydrogen atoms may be substituted by fluorine atoms, or a phenyl group, R⁴⁴⁴ is a C₁₋₆ linear or branched alkyl group wherein some or all of hydrogen atoms may be substituted by fluorine atoms, a phenyl group or a group of the formula: 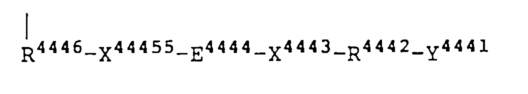 wherein each of R⁴⁴⁴⁶ and R⁴⁴⁴² which are independent of each other, is a C₁₋₆ linear or branched alkylene group, each of X⁴⁴⁴⁵ and X⁴⁴⁴³ is a covalent bond, an oxygen atom or a C₁₋₆ alkylene glycol group, E⁴⁴⁴⁴ is a -CONH- group or a bivalent group wherein the terminals derived from a diisocyanate selected from a saturated aliphatic type, an alicyclic type and an aromatic type, are a -CONH- group and a -NHCO- group, and Y⁴⁴⁴¹ is an acryloyloxy group, a methacryloyloxy group, a vinyl group or an allyl group, provided that each of X⁴⁴⁴⁵ and X⁴⁴⁴³ is a covalent bond when the adjacent E⁴⁴⁴⁴ is a -CONH- group, or an oxygen atom or a C₁₋₆ alkylene glycol group when the adjacent E⁴⁴⁴⁴ is a bivalent group derived from the isocyanate, and E⁴⁴⁴⁴ forms a urethane bond between the adjacent X⁴⁴⁴⁵ and X⁴⁴⁴³, provided that a case wherein all of R⁴⁴¹, R⁴⁴², R⁴⁴³, R⁴⁴⁴, R⁴⁴⁵ and R⁴⁴⁶ are simultaneously C₁₋₆ linear or branched alkyl groups wherein some or all of hydrogen atoms are substituted by fluorine atoms, or phenyl groups, is excluded, m441 is an integer of from 1 to 100, and m442 is an integer of from 0 to (100-(m441), provided that (m441)+(m442) is an integer of from 1 to 100, S⁴⁵ is a group of the formula: 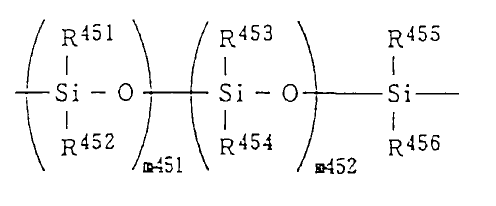 wherein each of R⁴⁵¹, R⁴⁵², R⁴⁵³, R⁴⁵⁵ and R⁴⁵⁶ which are independent of one another, is a C₁₋₆ linear or branched alkyl group wherein some or all of hydrogen atoms may be substituted by fluorine atoms, or a phenyl group, R⁴⁵⁴ is a C₁₋₆ linear branched alkyl group wherein some or all of hydrogen atoms may be substituted by fluorine atoms, a phenyl group or a group of the formula: 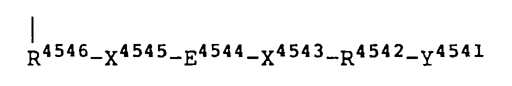 wherein each of R⁴⁵⁴⁶ and R⁴⁵⁴² which are independent of each other, is a C₁₋₆ linear branched alkylene group, each of X⁴⁵⁴⁵ and X⁴⁵⁴³ is a covalent bond, an oxygen atom or a C₁₋₆ alkylene glycol group, E⁴⁵⁴⁴ is a -CONH- group or a bivalent group wherein the terminals derived from a diisocyanate selected from a saturated aliphatic type, an alicyclic type and an aromatic type, are a -CONH- group and a -NHCO- group, Y⁴⁵⁴¹ is an acryloyloxy group, a methacryloyloxy group, a vinyl group or an allyl group, provided that each of X⁴⁵⁴⁵ and X⁴⁵⁴³ is a covalent bond when the adjacent E⁴⁵⁴⁴ is a -CONH- group, or an oxygen atom or a C₁₋₆ alkylene glycol group when the adjacent E⁴⁵⁴⁴ is a bivalent group derived from the diisocyanate, and E⁴⁵⁴⁴ forms a urethane bond between the adjacent X⁴⁵⁴⁵ and X⁴⁵⁴³, provided that a case wherein all of R⁴⁵¹, R⁴⁵², R⁴⁵³, R⁴⁵⁴, R⁴⁵⁵ and R⁴⁵⁶ are simultaneously C₁₋₆ linear or branched alkyl groups wherein some or all of hydrogen atoms are substituted by fluorine atoms, or phenyl groups, is excluded, m451 is an integer of from 1 to 100, and m452 is an integer of from 0 to (100-(m451), provided that (m451)+(m452) is an integer of from 1 to 100, U⁴⁶ is a group of the formula:

-R⁴⁶¹-X⁴⁶²-E⁴⁶³-X⁴⁶⁴-

wherein R⁴⁶¹ is a C₁₋₆ linear or branched alkylene group, X⁴⁶² is an oxygen atom, a C₁₋₆ alkylene glycol group or a group of the formula: 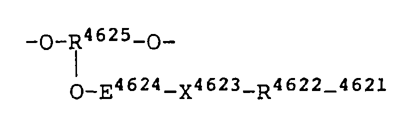 wherein R⁴⁶²⁵ is a C₁₋₆ trivalent hydrocarbon group, R⁴⁶²² is a C₁₋₆ linear or branched alkylene group, E⁴⁶²⁴ is a -CONH- group or a bivalent group wherein the terminals derived from a diisocyanate selected from a saturated aliphatic type, an alicyclic type and an aromatic type, are a -CONH- group and a -NHCO- group, X⁴⁶²³ is a covalent bond, an oxygen atom or a C₁₋₆ alkylene glycol group, and Y⁴⁶²¹ is an acryloyloxy group, a methacryloyloxy group, a vinyl group or an allyl group, provided that X⁴⁶²³ is a covalent bond when the adjacent E⁴⁶²⁴ is a -CONH- group, or an oxygen atom or a C₁₋₆ alkylene glycol group when the adjacent E⁴⁶²⁴ is a bivalent group derived from the isocyanate, and E⁴⁶²⁴ forms a urethane bond between the adjacent oxygen atom and X⁴⁶²³, E⁴⁶³ is a -CONH- group or a bivalent group wherein the terminals derived from a diisocyanate selected from a saturated aliphatic type, an alicyclic type and an aromatic type, are a -CONH- group and a -NHCO- group, X⁴⁶⁴ is a covalent bond, an oxygen atom or a C₁₋₆ alkylene glycol group, provided that X⁴⁶⁴ is a covalent bond when the adjacent E⁴⁶³ is a -CONH- group, or an oxygen atom or a C₁₋₆ alkylene glycol group when the adjacent E⁴⁶³ is a bivalent group derived from the diisocyanate, and E⁴⁶³ forms a urethane bond between the adjacent X⁴⁶² and X⁴⁶⁴, and A⁴⁷ is a group of the formula:

-R⁴⁷²-Y⁴⁷¹

wherein R⁴⁷² is a C₁₋₆ linear or branched alkylene group, and R⁴⁷¹ is an acryloyloxy group, a methacryloyloxy group, a vinyl group or an allyl group, and m46 is 0 or 1, m2 is 0 or 1, m4 is 0, 1, 2 or 3, and m5 is 0 or 1, provided that m2, m5 and m46 are all the same; such as an urethane bond-containing polysiloxane macromonomer (hereinafter referred to as macromonomer a) of the formula:

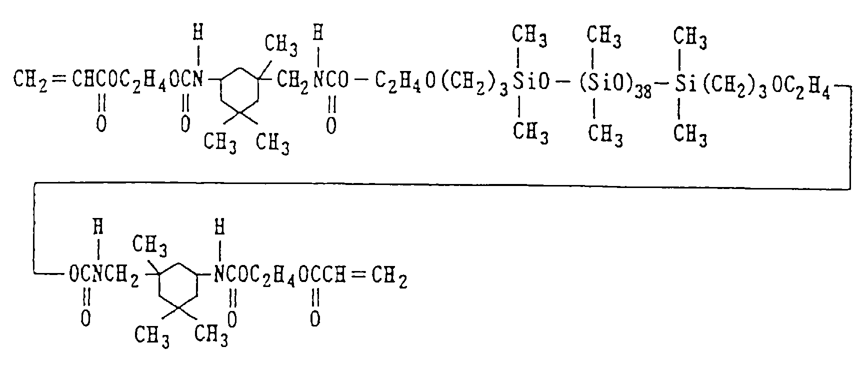

Such polysiloxane macromonomers may be used alone or in combination as a mixture of two or more of them. The content of such a polysiloxane macromonomer may be suitably adjusted depending upon the nature of the desired ocular lens material. For example, in a case where it is desired to obtain a low water-absorptive highly oxygen permeable ocular lens material, in order to obtain adequate effects for improving oxygen permeability and for reinforcing the mechanical strength, it is advisable to adjust the content of the polysiloxane macromonomer to a level of at least 2 wt%, preferably at least 5 wt%, more preferably at least 10 wt% of the total amount of the polymerizable components. Further, in order to avoid deterioration of the compatibility with other polymerizable components, it is advisable to adjust the content to a level of at most 30 wt%, preferably at most 20 wt%, more preferably at most 15 wt%, of the total amount of the polymerizable components.

For example, when it is desired to further improve the oxygen permeability of the resulting ocular lens material, a silicon-containing monomer other than the silicon-containing compound of the formula (I), such as a silicon-containing alkyl (meth)acrylate, a silicon-containing styrene derivative or an alkylvinyl silane, may be employed.

The silicon-containing alkyl (meth)acrylate may, for example, be an organopolysiloxane-containing alkyl (meth)acrylate, such as pentamethyldisiloxanylmethyl (meth)acrylate, trimethylsiloxydimethylsilylpropyl (meth)acrylate, methylbis(trimethylsiloxy)silylpropyl (meth)acrylate, tris(trimethylsiloxy)silylpropyl (meth)acrylate, mono[methylbis(trimethylsiloxy)siloxy]bis(trimethyl siloxy)silylpropyl (meth)acrylate, tris[methylbis(trimethylsiloxy)siloxy]silylpropyl (meth)acrylate, methyl[bis(trimethylsiloxy)]silylpropylglyceryl (meth)acrylate, tris(trimethylsiloxy)silylpropylglyceryl (meth)acrylate, mono[methylbis(trimethylsiloxy)siloxy]bis(trimethyl siloxy)silylpropylglyceryl (meth)acrylate, trimethylsilylethyltetramethyldisiloxanylpropylglyceryl (meth)acrylate, trimethylsilylmethyl (meth)acrylate, trimethylsilylpropyl (meth)acrylate, trimethylsilylpropylglyceryl (meth)acrylate, pentamethyldisiloxanylpropylglyceryl (meth)acrylate, methylbis(trimethylsiloxy)silylethyltetramethyl disiloxanylmethyl (meth)acrylate, tetramethyltriisopropylcyclotetrasiloxanylpropyl (meth)acrylate, tetramethyltriisopropylcyclotetrasiloxybis(trimethyl siloxy)silylpropyl (meth)acrylate or trimethylsiloxydimethylsilylpropyl (meth)acrylate.

The above silicon-containing styrene derivative may, for example, be an organopolysiloxane-containing styrene derivative of the formula (VI): 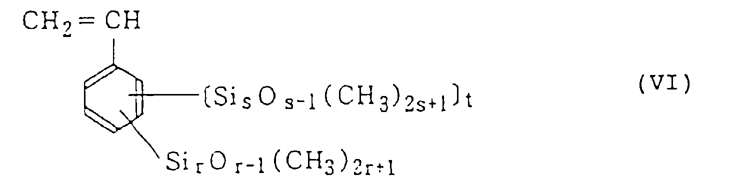 wherein s is an integer of from 1 to 15, t is 0 or 1, and r is an integer of from 1 to 15. In the organopolysiloxane-containing styrene derivative of the formula (VI), if s or r is an integer of 16 or higher, the synthesis or purification tends to be difficult, and the hardness of the resulting ocular lens material tends to be low. Further, if t is an integer of 2 or higher, the synthesis of such an organopolysiloxane-containing styrene derivative tends to be difficult.

Typical examples of the styrene derivative of the above formula (VI) include tris(trimethylsiloxy)silyl styrene, bis(trimethylsiloxy)methylsilyl styrene, dimethylsilyl styrene, trimethylsilyl styrene, tris(trimethylsiloxy)siloxanyldimethylsilyl styrene, [bis(trimethylsiloxy)methylsiloxanyl]dimethylsilyl styrene, pentamethyldisiloxanyl styrene, heptamethyltrisiloxanyl styrene, nonamethyltetrasiloxanyl styrene, pentadecamethylheptasiloxanyl styrene, heneicosamethyldecasiloxanyl styrene, heptacosamethyltridecasiloxanyl styrene, hentriacontamethylpentadecasiloxanyl styrene, trimethylsiloxypentamethyldisiloxymethylsilyl styrene, tris(pentamethyldisiloxy)silyl styrene, (tristrimethylsiloxy)siloxanylbis(trimethylsiloxy)silyl styrene, bis(heptamethyltrisiloxy)methylsilyl styrene, tris(methylbistrimetnylsiloxysiloxy)silyl styrene, trimethylsiloxybis(tristrimethylsiloxysiloxy)silyl styrene, heptakis(trimethylsiloxy)trisiloxanyl styrene, tris(tristrimethylsiloxysiloxy)silyl styrene, (tristrimethylsiloxyhexamethyl)tetrasiloxy(tristrimethyl siloxy)siloxytrimethylsiloxysilyl styrene, nonakis(trimethylsiloxy)tetrasiloxanyl styrene, bis(tridecamethylhexasiloxy)methylsilyl styrene, heptamethylcyclotetrasiloxanyl styrene, heptamethylcyclotetrasiloxybis(trimethylsiloxy)silyl styrene, and tripropyltetramethylcyclotetrasiloxanyl styrene.

The above alkylvinyl silane may, for example, be trimethylvinyl silane.

Among the above silicon-containing monomers, tris(trimethylsiloxy)silylpropyl (meth)acrylate and tris(trimethylsiloxy)silyl styrene are particularly preferred, since they are excellent in the compatibility with other polymerizable components, and they have large effects for improving the oxygen permeability of the resulting ocular lens material.

Such silicon-containing monomers may be used alone or in combination as a mixture of two or more of them. The content of such a silicon-containing monomer may suitably be adjusted depending upon the nature of the desired ocular lens material. For example, when it is desired to obtain a low water-absorptive highly oxygen permeable ocular lens material, in order to obtain adequate effects for improving the oxygen permeability, it is advisable to adjust the content of the silicon-containing monomer to a level of at least 5 wt%, preferably at least 10 wt%, more preferably at least 20 wt%, of the total amount of the polymerizable components. Further, in order to avoid deterioration of the compatibility with other polymerizable components, it is advisable to adjust the content to a level of at most 70 wt%, preferably at most 60 wt%, more preferably at most 50 wt%, of the total amount of the polymerizable components.

When it is desired to further improve the hydrophilic property of the resulting ocular lens material and to impart water absorptivity to the ocular lens material, a hydrophilic monomer having a hydroxyl group, an amide group, a carboxyl group, an amino group, a glycol residue, a pyrrolidone structure or a morpholine structure, may, for example, be used.

The above hydrophilic monomer may, for example, be a hydroxyalkyl (meth)acrylate such as 2-hydroxyethyl (meth)acrylate, hydroxybutyl (meth)acrylate or hydroxypropyl (meth)acrylate; an (alkyl)aminoalkyl (meth)acrylate such as 2-dimethylaminoethyl (meth)acrylate or 2-butylaminoethyl (meth)acrylate; an alkyl (meth)acrylamide such as N,N-dimethyl (meth)acrylamide; a polyglycol mono(meth)acrylate such as propylene glycol mono(meth)acrylate; vinyl pyrrolidone; (meth)acrylic acid; maleic anhydride, fumaric acid; a fumaric acid derivative; amino styrene; hydroxy styrene, or a morpholino compound of the formula (VII): 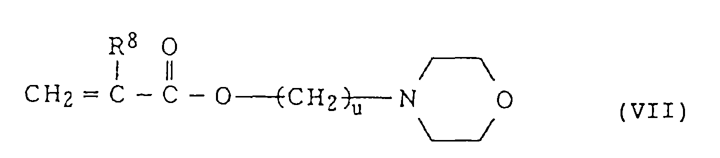 wherein R⁸ is a hydrogen atom or a methyl group, and u is an integer of from 1 to 12.

In the formula (VII) representing the above morpholino compound, u is an integer of from 1 to 12, and if u is an integer larger than 12, the alkylene chain will be long, and the hydrophobic moiety tends to increase too much, whereby such a morpholino compound tends to hardly provide adequate effects for imparting a hydrophilic property.

Typical examples of such a morpholino compound include morpholinomethyl (meth)acrylate and morpholinoethyl (meth)acrylate.

Among the above hydrophilic monomers, an alkyl (meth)acryloamide, (meth)acrylic acid and a hydroxyalkyl (meth)acrylate are particularly preferred, since they are excellent in the compatibility with other polymerizable components, and they have large effects for improving the hydrophilic property of the resulting ocular lens material.

Further, the above morpholino compound is preferred, since it not only imparts a proper hydrophilic property to the resulting ocular lens material but is excellent in the compatibility with a hydrophobic component, particularly with a silicon-containing hydrophobic component, so that even when it is polymerized in admixture with such a hydrophobic component, the resulting polymer will be excellent in transparency without turbidity.

Further, when such a morpholino compound is used, a proper hydrophilic property will be imparted to the resulting ocular lens material. Accordingly, even if the resulting ocular lens material is, for example, a water non-absorptive material, a hydrophobic stain such as lipid tends to scarcely attach. Further, by properly adjusting the content of such a morpholino compound, the material may be made water-absorptive.

The above hydrophilic monomers may be used alone or in combination as a mixture of two or more of them. The content of such a hydrophilic monomer may suitably be adjusted depending upon the nature of the desired ocular lens material. For example, when it is desired to obtain a highly oxygen permeable ocular lens material, in order to obtain adequate effects for improving the hydrophilic property or for imparting the water absorptivity, it is advisable to adjust the content of the hydrophilic monomer to a level of at least 5 wt%, preferably at least 10 wt%, more preferably at least 20 wt%, of the total amount of the polymerizable components. Further, in order to avoid an excessive increase in the water absorptivity of the resulting ocular lens material, it is advisable to adjust the content to a level of at most 80 wt%, preferably at most 75 wt%, more preferably at most 70 wt%, of the total amount of the polymerizable components.

Further, when it is desired to improve the mechanical strength or durability of the resulting ocular lens material and to impart waster resistance and solvent resistance to the ocular lens material, it is preferred to employ a crosslinkable monomer which is a polyfunctional polymerizable compound having at least two copolymerizable unsaturated double bonds.

Such a crosslinkable monomer may, for example, be ethylene glycol di(meth)acrylate, diethylene glycol di(meth)acrylate, triethylene glycol di(meth)acrylate, propylene glycol di(meth)acrylate, dipropylene glycol di(meth)acrylate, allyl (meth)acrylate, vinyl (meth)acrylate, trimethylolpropane tri(meth)acrylate, methacryloyloxyethyl acrylate, divinylbenzene, diallyl phthalate, diallyl adipate, triallyl isocyanurate, α-methylene-N-vinylpyrrolidone, 4-vinylbenzyl (meth)acrylate, 3-vinylbenzyl (meth)acrylate, 2,2-bis(p-(meth)acryloyloxyphenyl)hexafluoropropane, 2,2-bis(m-(meth)acryloyloxyphenyl)hexafluoropropane, 2,2-bis(o-(meth)acryloyloxyphenyl)hexafluoropropane, 2,2-bis(p-(meth)acryloyloxyphenyl)propane, 2,2-bis(m-(meth)acryloyloxyphenyl)propane, 2,2-bis(o-(meth)acryloyloxyphenyl)propane, 1,4-bis(2-(meth)acryloyloxyhexafluoroisopropyl)benzene, 1,3-bis(2-(meth)acryloyloxyhexafluoroisopropyl)benzene, 1,2-bis(2-(meth)acryloyloxyhexafluoroisopropyl)benzene, 1,4-bis(2-(meth)acryloyloxyisopropyl)benzene, 1,3-bis(2-(meth)acryloyloxyisopropyl)benzene, or 1,2-bis(2-(meth)acryloyloxyisopropyl)benzene.

Among the above crosslinkable monomers, ethylene glycol di(meth)acrylate is particularly preferred, since it is excellent in the compatibility with other polymerizable components, and it has large effects for improving the mechanical strength of the resulting ocular lens material.

The above crosslinkable monomers may be used alone or in combination as a mixture of two or more of them. The content of such a crosslinkable monomer may be suitably adjusted depending upon the nature of the desired ocular lens material. For example, when it is desired to obtain a low water-absorptive highly oxygen permeable ocular lens material, in order to obtain adequate effects for improving the mechanical strength, durability, water resistance, solvent resistance, etc., it is advisable to adjust the content of the crosslinkable monomer to a level of at least 0.01 wt%, preferably at least 0.05 wt%, more preferably at least 0.1 wt%, of the total amount of the polymerizable components. Further, in order to avoid possible brittleness of the resulting ocular lens material, it is advisable to adjust the content to a level of at most 10 wt%, preferably at most 5 wt%, more preferably at most 3 wt%, of the total amount of the polymerizable components.

Further, when it is desired to impart deposit resistance to the resulting ocular lens material, it is preferred to employ a fluorine-containing monomer which is a polymerizable compound having some of hydrogen atoms of a hydrocarbon group substituted by fluorine atoms.

Such a fluorine-containing monomer may, for example, be a monomer of the formula (VIII):

CH₂=CR⁹COOCₐH_{(2a-b-c+1)}F_{b}(OH)_{c} (VIII)

wherein R⁹ is a hydrogen atom or a methyl group, a is an integer of from 1 to 15, b is a integer of from 1 to (2a+1), and c is an integer of from 0 to 2.

Typical examples of the monomer of the above formula (VIII) include 2,2,2-trifluoroethyl (meth)acrylate, 2,2,3,3-tetrafluoropropyl (meth)acrylate, 2,2,3,3-tetrafluoro-t-pentyl (meth)acrylate, 2,2,3,4,4,4-hexafluorobutyl (meth)acrylate, 2,2,3,4,4,4-hexafluoro-t-hexyl (meth)acrylate, 2,3,4,5,5,5-hexafluoro-2,4-bis(trifluoromethyl)pentyl (meth)acrylate, 2,2,3,3,4,4-hexafluorobutyl (meth)acrylate, 2,2,2,2',2',2'-hexafluoroisopropyl (meth)acrylate, 2,2,3,3,4,4,4-heptafluorobutyl (meth)acrylate, 2,2,3,3,4,4,5,5-octafluoropentyl (meth)acrylate, 2,2,3,3,4,4,5,5,5-nonafluoropentyl (meth)acrylate, 2,2,3,3,4,4,5,5,6,6,7,7-dodecafluoroheptyl (meth)acrylate, 3,3,4,4,5,5,6,6,7,7,8,8-dodecafluorooctyl (meth)acrylate, 3,3,4,4,5,5,6,6,7,7,8,8,8-tridecafluorooctyl (meth)acrylate, 2,2,3,3,4,4,5,5,6,6,7,7,7-tridecafluoroheptyl (meth)acrylate, 3,3,4,4,5,5,6,6,7,7,8,8,9,9,10,10-hexadecafluorodecyl (meth)acrylate, 3,3,4,4,5,5,6,6,7,7,8,8,9,9,10,10,10-heptadecafluorodecyl (meth)acrylate, 3,3,4,4,5,5,6,6,7,7,8,8,9,9,10,10,11,11-octadecafluoroundecyl (meth)acrylate, 3,3,4,4,5,5,6,6,7,7,8,8,9,9,10,10,11,11,11-nonadecafluoroundecyl (meth)acrylate, 3,3,4,4,5,5,6,6,7,7,8,8,9,9,10,10,11,11,12,12-eicosafluorododecyl (meth)acrylate, 2-hydroxy-4,4,5,5,6,7,7,7-octafluoro-6-trifluoromethylheptyl (meth)acrylate, 2-hydroxy-4,4,5,6,6,7,7,8,9,9,9-dodecafluoro-8-trifluoromethylnonyl (meth)acrylate and 2-hydroxy-4,4,5,5,6,6,7,7,8,8,9,9,10,11,11,11-hexadecafluoro-10-trifluoromethylundecyl (meth)acrylate. Among the above fluorine-containing monomers, 2,2,2-trifluoroethyl (meth)acrylate and 2,2,2,2',2',2'-hexafluoroisopropyl (meth)acrylate are particularly preferred, since they have large effects for improving the deposit resistance of the resulting ocular lens material.

The above fluorine-containing monomers may be used alone or in combination as a mixture of two or more of them. The content of such a fluorine-containing monomer may suitably be adjusted depending upon the nature of the desired ocular lens material. For example, when it is desired to obtain a low water-absorptive highly oxygen permeable ocular lens material, in order to obtain adequate effects for improving the deposit resistance, it is advisable to adjust the content of the fluorine-containing monomer to a level of at least 1 wt%, preferably at least 3 wt%, more preferably at least 5 wt%, of the total amount of the polymerizable components. Further, in order to avoid deterioration of the compatibility with other polymerizable components, it is advisable to adjust the content to a level of at most 30 wt%, preferably at most 20 wt%, more preferably at most 15 wt%, of the total amount of the polymerizable components.

Further, to impart hardness or softness by adjusting the hardness of the resulting ocular lens material, a hardness-adjusting monomer such as an alkyl (meth)acrylate or an alkyl styrene which is a polymerizable compound having an alkyl group, or styrene, may, for example, be used.

The above alkyl (meth)acrylate may, for example, be a linear, branched or cyclic alkyl (meth)acrylate, such as methyl (meth)acrylate, ethyl (meth)acrylate, isopropyl (meth)acrylate, n-propyl (meth)acrylate, isobutyl (meth)acrylate, n-butyl (meth)acrylate, 2-ethylhexyl (meth)acrylate, n-octyl (meth)acrylate, n-decyl (meth)acrylate, n-dodecyl (meth)acrylate, t-butyl (meth)acrylate, pentyl (meth)acrylate, t-pentyl (meth)acrylate, hexyl (meth)acrylate, heptyl (meth)acrylate, nonyl (meth)acrylate, stearyl (meth)acrylate, cyclopentyl (meth)acrylate or cyclohexyl (meth)acrylate; an alkoxyalkyl (meth)acrylate such as 2-ethoxyethyl (meth)acrylate, 3-ethoxypropyl (meth)acrylate, 2-methoxyethyl (meth)acrylate or 3-methoxypropyl (meth)acrylate; or an alkylthioalkyl (meth)acrylate such as ethylthioethyl (meth)acrylate or methythioethyl (meth)acrylate.

The above alkyl styrene may, for example, be α-methyl styrene; an alkyl styrene such as methyl styrene, ethyl styrene, propyl styrene, butyl styrene, t-butyl styrene, isobutyl styrene or pentyl styrene; or an alkyl α-methyl styrene such as methyl-α-methyl styrene, ethyl-α-methyl styrene, propyl-α-methyl styrene, butyl-α-methyl styrene, t-butyl-α-methyl styrener isobutyl-α-methyl styrene or pentyl-α-methyl styrene.

When it is desired to obtain, for example, a soft contact lens, it is preferred to employ, among such hardness-adjusting monomers, the one which, when formed into a homopolymer, will have a glass transition temperature (hereinafter referred to as Tg) of at most 40°C. Further, when it is desired to obtain a hard contact lens, it is preferred to employ the one which, when formed into a homopolymer, will have a Tg of higher than 40°C. Among them, an alkyl (meth)acrylate and an alkyl styrene are particularly preferred, since they are excellent in compatibility or copolymerizability with other polymerizable components.

The above hardness-adjusting monomers may be used alone or in combination as a mixture of two or more of them. The content of such a hardness-adjusting monomer may suitably be adjusted depending upon the nature of the desired ocular lens material. For example, when it is desired to obtain a low water-absorptive highly oxygen permeable ocular lens material, in order to obtain adequate effects for adjusting the hardness, it is advisable to adjust the content of the hardness-adjusting monomer to a level of at least 1 wt%, preferably at least 3 wt%, more preferably at least 5 wt%, of the total amount of the polymerizable components. Further, to avoid a possibility such that the content of other polymerizable components such as a silicon-containing compound will be relatively so small that no adequate effects for improving the oxygen permeability, the hydrophilic property or the transparency will be obtained, it is advisable to adjust the content to a level of at most 80 wt%, preferably at most 75 wt%, more preferably at most 70 wt%, of the total amount of the polymerizable components.

Further, to impart ultraviolet absorptivity to the resulting ocular lens material or to color the ocular lens material, a polymerizable ultraviolet absorber, a polymerizable dyestuff or a polymerizable ultraviolet absorbing dyestuff may, for example, be used.

Specific examples of the polymerizable ultraviolet absorber include benzophenone type polymerizable ultraviolet absorbers such as 2-hydroxy-4-(meth)acryloyloxybenzophenone, 2-hydroxy-4-(meth)acryloyloxy-5-tert-butylbenzophenone, 2-hydroxy-4-(meth)acryloyloxy-2',4'-dichlorobenzophenone and 2-hydroxy-4-(2'-hydroxy-3'-(meth)acryloyloxypropoxy)benzophenone; benzotriazole type polymerizable ultraviolet absorbers such as 2-(2'-hydroxy-5'-(meth)acryloyloxyethylphenyl)-2H-benzotriazole, 2-(2'-hydroxy-5'-(meth)acryloyloxyethylphenyl)-5-chloro-2H-benzotriazole, 2-(2'-hydroxy-5'-(meth)acryloyloxypropylphenyl)-2H-benzotriazole and 2-(2'-hydroxy-5'-(meth)acryloyloxypropyl-3'-tert-butylphenyl)-5-chloro-2H-benzotriazole; salicylic acid derivative-type polymerizable ultraviolet absorbers such as phenyl 2-hydorxy-4-(meth)acryloyloxymethylbenzoate; and other polymerizable ultraviolet absorbers such as methyl 2-cyano-3-phenyl-3-(3'-(meth)acryloyloxyphenyl)propenoate. These polymerizable ultraviolet absorbers may be used alone or in combination as a mixture of two or more of them.

Specific examples of the polymerizable dyestuff include azo type polymerizable dyestuffs such as 1-phenylazo-4-(meth)acryloyloxynaphthalene, 1-phenylazo-2-hydroxy-3-(meth)acryloyloxynaphthalene, 1-naphthylazo-2-hydroxy-3-(meth)acryloyloxynaphthalene, 1-(α-anthrylazo)-2-hydroxy-3-(meth)acryloyloxynaphthalene, 1-((4'-(phenylazo)phenyl)azo)-2-hydroxy-3-(meth)acryloyloxynaphthalene, 1-(2',4'-xylylazo)-2-(meth)acryloyloxynaphthalene, 1-(o-tolylazo)-2-(meth)acryloyloxynaphthalene, 2-(m-(meth)acryloylamideanilino)-4,6-bis(1'-(o-tolylazo)-2'-naphthylamino)-1,3,5-triazine, 2-(m-vinylanilino)-4-((4'-nitrophenylazo)-anilino)-6-chloro-1,3,5-triazine, 2-(1'-(o-tolylazo)-2'-naphthyloxy-4-(m-vinylanilino)-6-chloro-1,3,5-triazine, 2-(p-vinylanilino)-4-(1'-(o-tolylazo)-2'-naphthylamino)-6-chloro-1,3,5-triazine, N-(1'-(o-tolylazo)-2'-naphthyl)-3-vinylphthalic acid monoamide, N-(1'-(o-tolylazo)-2'-naphthyl)-6-vinylphthalic acid monoamide, 3-vinylphthalic acid-(4'-(p-sulfophenylazo)-1'-naphthyl)monoester, 6-vinylphthalic acid-(4'-(p-sulfophenylazo)-1'-naphthyl)monoester, 3-(meth)acryloylamide-4-phenylazophenol, 3-(meth)acryloylamide-4-(8'-hydroxy-3',6'-disulfo-1'-naphthylazo)phenol, 3-(meth)acryloylamide-4-(1'-phenylazo-2'-naphthylazo)phenol, 3-(meth)acryloylamide-4-(p-tolylazo)phenol, 2-amino-4-(m-(2'-hydroxy-1'-naphthylazo)anilino)-6-isopropenyl-1,3,5-triazine, 2-amino-4-(N-methyl-p-(2'-hydroxy-1'-naphthylazo)anilino)- 6-isopropenyl-1,3,5-triazine, 2-amino-4-(m-(4'-hydroxy-1'-phenylazo)anilino)-6-isopropenyl-1,3,5-triazine, 2-amino-4-(N-methyl-p-(4'-hydroxyphenylazo)anilino)-6-isopropenyl-1,3,5-triazine, 2-amino-4-(m-(3'-methyl-1'-phenyl-5'-hydroxy-4'-pyrazolylazo)anilino)-6-isopropenyl-1,3,5-triazine, 2-amino-4-(N-methyl-p-(3'-methyl-1'-phenyl-5'-hydroxy-4'-pyrazolylazo)anilino)-6-isopropenyl-1,3,5-triazine, 2-amino-4-(p-phenylazoanilino)-6-isopropenyl-1,3,5-triazine and 4-phenylazo-7-(meth)acryloylamide-1-naphthol; anthraquinone type polymerizable dyestuffs such as 1,5-bis((meth)acrylcylamino)-9,10-anthraquinone, 1-(4'-vinylbenzoylamide)-9,10-anthraquinone, 4-amino-1-(4'-vinylbenzoylamide)-9,10-anthraquinone, 5-amino-1-(4'-vinylbenzoylamide)-9,10-anthraquinone, 8-amino-1-(4'-vinylbenzoylamide)-9,10-anthraquinone, 4-nitro-1-(4'-vinylbenzoylamide)-9,10-anthraquinone, 4-hydroxy-1-(4'-vinylbenzoylamide)-9,10-anthraquinone, 1-(3'-vinylbenzoylamide)-9,10-anthraquinone, 1-(2'-vinylbenzoylamide)-9,10-anthraquinone, 1-(4'-isopropenylbenzoylamide)-9,10-anthraquinone, 1-(3'-isopropenylbenzoylamide)-9,10-anthraquinone, 1-(2'-isopropenylbenzoylamide)-9,10-anthraquinone, 1,4-bis-(4'-vinylbenzoylamide)-9,10-anthraquinone, 1,4-bis-(4'-isopropenylbenzoylamide)-9,10-anthraquinone, 1,5-bis-(4'-vinylbenzoylamide)-9,10-anthraquinone, 1,5-bis-(4'-isopropenylbenzoylamide)-9,10-anthraquinone, 1-methylamino-4-(3'-vinylbenzoylamide)-9,10-anthraquinone, 1-methylamino-4-(4'-vinylbenzoyloxyethylamino)-9,10-anthraquinone, 1-amino-4-(3'-vinylphenylamino)-9,10-anthraquinone-2-sulfonic acid, 1-amino-4-(4'-vinylphenylamino)-9,10-anthraquinone-2-sulfonic acid, 1-amino-4-(2'-vinylbenzylamino)-9,10-anthraquinone-2-sulfonic acid, 1-amino-4-(3'-(meth)acryloylaminophenylamino)-9,10-anthraquinone-2-sulfonic acid, 1-amino-4-(3'-(meth)acryloylaminobenzylamino)-9,10-anthraquinone-2-sulfonic acid, 1-(β-ethoxycarbonylallylamino)-9,10-anthraquinone, 1-(β-carboxyallylamino)-9,10-anthraquinone, 1,5-di-(β-carboxyallylamino)-9,10-anthraquinone, 1-(β-isopropoxycarbonylallylamino)-5-benzoylamide-9,10-anthraquinone, 2-(3'-(meth)acryloylamide-anilino)-4-(3'-(3''-sulfo-4''-aminoanthraquinon-1''-yl)amino-anilino)-6-chloro-1,3,5-triazine, 2-(3'-(meth)acryloylamide-anilino)-4-(3'-(3''-sulfo-4''-aminoanthraquinon-1''-yl)amino-anilino)-6-hydrazino-1,3,5-triazine, 2,4-bis-((4''-methoxyanthraquinon-1''-yl)amino)-6-(3'-vinylanilino)-1,3,5-triazine and 2-(2'-vinylphenoxy)-4-(4'-(3''-sulfo-4''-aminoanthraquinon-1''-yl-amino)anilino)-6-chloro-1,3,5-triazine; nitro type polymerizable dyestuffs such as o-nitroanilinomethyl (meth)acrylate; and phthalocyanine type polymerizable dyestuffs such as (meth)acryloyl-modified tetraamino copper phthalocyanine and (meth)acryloyl-modified (dodecanoyl-modified tetraamino copper phthalocyanine). These polymerizable dyestuffs may be used alone or in combination as a mixture of two or more of them.

Specific examples of the polymerizable ultraviolet absorbing dyestuff include benzophenone type polymerizable ultraviolet absorbing dyestuffs such as 2,4-dihydroxy-3-(p-styrenoazo)benzophenone, 2,4-dihydroxy-5-(p-styrenoazo)benzophenone, 2,4-dihydroxy-3-(p-(meth)acryloyloxymethylphenylazo)benzophenone, 2,4-dihydroxy-5-(p-(meth)acryloyloxymethylphenylazo)-benzophenone, 2,4-dihydroxy-3-(p-(meth)acryloyloxyethylphenylazo)benzophenone, 2,4-dihydroxy-5-(p-(meth)acryloyloxyethylphenylazo)-benzophenone, 2,4-dihydroxy-3-(p-(meth)acryloyloxypropylphenylazo)benzophenone, 2,4-dihydroxy-5-(p-(meth)acryloyloxypropylphenylazo)-benzophenone, 2,4-dihydroxy-3-(o-(meth)acryloyloxymethylphenylazo)benzophenone, 2,4-dihydroxy-5-(o-(meth)acryloyloxymethylphenylazo)benzophenone, 2,4-dihydroxy-3-(o-(meth)acryloyloxyethylphenylazo)benzophenone, 2,4-dihydroxy-5-(o-(meth)acryloyloxyethylphenylazo)benzophenone, 2,4-dihydroxy-3-(o-(meth)acryloyloxypropylphenylazo)benzophenone, 2,4-dihydroxy-5-(o-(meth)acryloyloxypropylphenylazo) benzophenone, 2,4-dihydroxy-3-(p-(N,N-di(meth)acryloyloxyethylamino)phenylazo)benzophenone, 2,4-dihydroxy-5-(p-(N,N-di(meth)acryloyloxyethylamino)phenylazo)benzophenone, 2,4-dihydroxy-3-(o-(N,N-di(meth)acryloyloxyethylamino)phenylazo)benzophenone, 2,4-dihydroxy-5-(o-(N,N-di(meth)acryloylethylamino)phenylazo)benzophenone, 2,4-dihydroxy-3-(p-(N-ethyl-N-(meth)acryloyloxyethylamino)phenylazo)benzophenone, 2,4-dihydroxy-5-(p-(N-ethyl-N-(meth)acryloyloxyethylamino)phenylazo)benzophenone, 2,4-dihydroxy-3-(o-(N-ethyl-N-(meth)acryloyloxyethylamino)phenylazo)benzophenone, 2,4-dihydroxy-5-(o-(N-ethyl-N-(meth)acryloyloxyethylamino)phenylazo)benzophenone, 2,4-dihydroxy-3-(p-(N-ethyl-N-(meth)acryloylamino)phenylazo)benzophenone, 2,4-dihydroxy-5-(p-(N-ethyl-N-(meth)acryloylamino)phenylazo)benzophenone, 2,4-dihydroxy-3-(o-(N-ethyl-N-(meth)acryloylamino)phenylazo)benzophenone and 2,4-dihydroxy-5-(o-(N-ethyl-N-(meth)acryloylamino)phenylazo)benzophenone; and benzoic acid type polymerizable ultraviolet absorbing dyestuffs such as phenyl 2-hydroxy-4-(p-styrenoazo)benzoate. These polymerizable ultraviolet absorbing dyestuffs may be used alone or in combination as a mixture of two or more of them.

The content of the above polymerizable ultraviolet absorber, polymerizable dyestuff and polymerizable ultraviolet absorbing dyestuff, may suitably be adjusted depending upon the nature of the desired ocular lens material. However, it is preferably at most 3 wt%, more preferably from 0.1 to 2 wt%, of the total amount of the polymerizable components, since it is substantially influenced by the thickness of the lens. If the content exceeds the above upper limit, the physical properties of the ocular lens material, such as mechanical strength, tend to deteriorate. Further, in consideration of the toxicity of the ultraviolet absorber or dyestuff, such an excessive content tends to be unsuitable as a material for ocular lenses such as contact lenses which are in direct contact with living tissues or intraocular lenses embedded in living bodies. Further, particularly in the case of a dyestuff, if the content is too much, the color of the lens tends to be so deep that the transparency decreases, and visible rays tend to be hardly transmitted through the lens.

In the present invention, among the above polymerizable monomers, one or more monomers other than polysiloxane macromonomers, may be selected and formed into a macromonomer, which is then incorporated, as one of the polymerizable monomers, to the polymerizable components.

The polymerizable components comprising the silicon-containing compound and the optional polymerizable monomers, are suitably adjusted and subjected to polymerization depending upon the use of the desired ocular lens such as a contact lens or an intraocular lens.

In the present invention, the polymerization can be carried out by a conventional method by adjusting the amounts of the polymerizable components comprising the silicon-containing compound and the optional polymerizable monomers within the ranges of the above-mentioned respective contents and adding e.g. a radical polymerization initiator thereto, to obtain a polymer.

The conventional method is, for example, a method wherein a radial polymerization initiator is added, followed by gradual heating within a temperature range of from room temperature to about 130°C (heat polymerization) or by irradiation of an electromagnetic wave such as a microwave, ultraviolet rays or a radiation (such as γ-rays). In the case of heat polymerization, the temperature may be raised stepwise. The polymerization may be carried out by a bulk polymerization method or a solution polymerization method employing e.g. a solvent, or by other methods.

Typical examples of the above-mentioned radical polymerization initiator include azobisisobutyronitrile, azobisodimethylvaleronitrile, benzoyl peroxide, t-butyl hydroperoxide and cumene hydroperoxide. These initiators may be used alone or in combination as a mixture of two or more of them.

In a case where the polymerization is carried out by means of e.g. light rays, it is preferred to further incorporate a photopolymerization initiator or a sensitizer. The amount of such a photopolymerization initiator or a sensitizer is usually from 0.001 to 2 parts by weight, preferably from 0.01 to 1 part by weight, per 100 parts by weight of the total amount of the polymerizable components.

To shape the ocular lens material of the present invention into an ocular lens such as a contact lens or an intraocular lens, conventional shaping methods which are commonly used in this field can be employed. Such conventional methods include, for example, a cutting method and a cast molding method. The cutting method is method wherein, after carrying out polymerization in a suitable mold or container to obtain a base material (polymer) of a rod, block or plate shape, such a base material is processed into a desired shape by mechanical processing such as cutting, grinding or polishing. The cast molding method is a method wherein a mold corresponding to the shape of a desired ocular lens, is prepared, and polymerization of the above-mentioned polymerizable components is carried out in this mold to obtain a molded product, which may further be subjected to mechanical finishing as the case requires. Further, a combination of such a cutting method and a cast molding method, may also be employed.

When it is desired to obtain the ocular lens material of the present invention as a soft material at a temperature around room temperature and such a material is shaped into an ocular lens, it is usually preferred to employ a cast molding method as the shaping method. Such a casting method may, for example, be a spin casting method or a static casting method.

Apart from these methods, a method may preferably be applied to the present invention wherein a monomer capable of forming a hard polymer is impregnated to a soft ocular lens material, followed by polymerization of the monomer to harden the entire material, which is then subjected to cutting, grinding and polishing to obtain a shaped product having a desired shape, and then the hard polymer is removed from the shaped product to obtain a shaped final product (an ocular lens) made of the soft material (JP-A-62-2780241, JP-A-1-11854).

Further, to prepare an intraocular lens, the lens and the support for the lens may separately be prepared, and they may be later bonded to each other. Otherwise, the support portion may be simultaneously (integrally) molded with the lens.

The ocular lens material of the present invention thus obtained, is made of a polymer obtained by polymerizing the polymerizable components containing the silicon-containing compound of the formula (I), and it is excellent particularly in oxygen permeability and transparency and has a proper hydrophilic property, whereby it is suitable for use as an ocular lens such as a contact lens, an intraocular lens and an artificial cornea.

Now, the ocular lens material of the present invention will be described in further detail with reference to Examples. However, it should be understood that the present invention is by no means restricted to such specific Examples.

### EXAMPLE 1

### Synthesis of N-tris(trimethylsiloxy)silylpropylmethacryl oxyethyl carbamate

Into a three-necked flask having a capacity of 1ℓ and equipped with a reflux condenser, a thermometer, a dropping funnel and a stirrer, 50 g (about 0.4 mol) of 2-hydroxyethyl methacrylate and 1.2 g of dibutyltin dilaurate were charged in a state not to allow moisture to enter, and while maintaining the temperature at a level of from 30 to 40°C, 76 g (about 0.2 mol) of tris(trimethylsiloxy)silylpropyl isocyanate was dropwise added thereto, followed by stirring at the same temperature for 16 hours.

Then, unreacted 2-hydroxyethyl methacrylate was removed by washing with water in a separating funnel, and the product was vacuum-dried to obtain 70 g of a colorless transparent liquid product (yield: 84%).

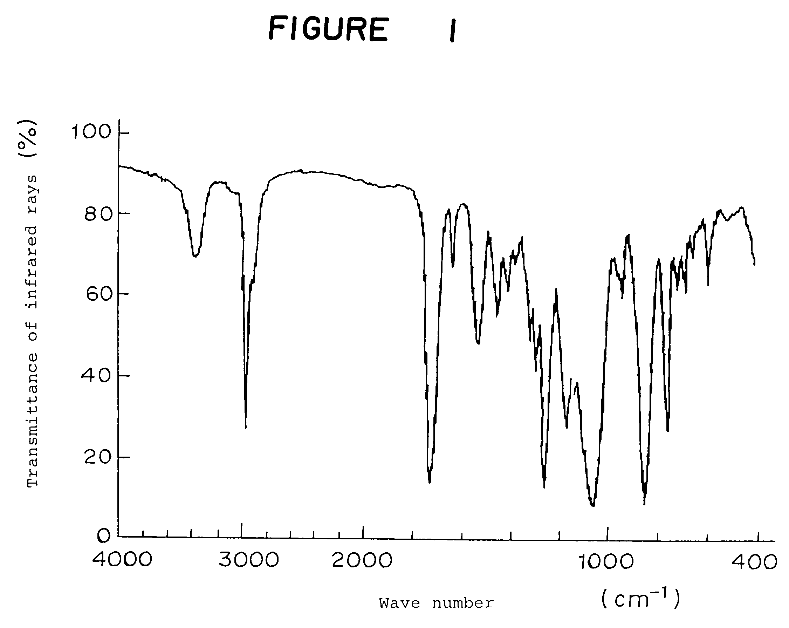

With respect to the obtained product, the infrared absorption spectrum was measured by a KBr liquid film method by means of FT-IR8300 manufactured by Nippon Bunko Kogyo K.K. The results are shown in Figure 1 as the transmittance of infrared rays.

The results shown in Figure 1 indicate that the absorption attributable to a urethane bond (-OCONH-) was observed in the vicinity of 3,400 cm⁻¹ and 1,750 cm⁻¹, the absorption attributable to Si-O-Si was observed in the vicinity of 1,058 cm⁻¹, and the absorption attributable to (CH₃)-SiO- was observed in the vicinity of 843 cm⁻¹ .

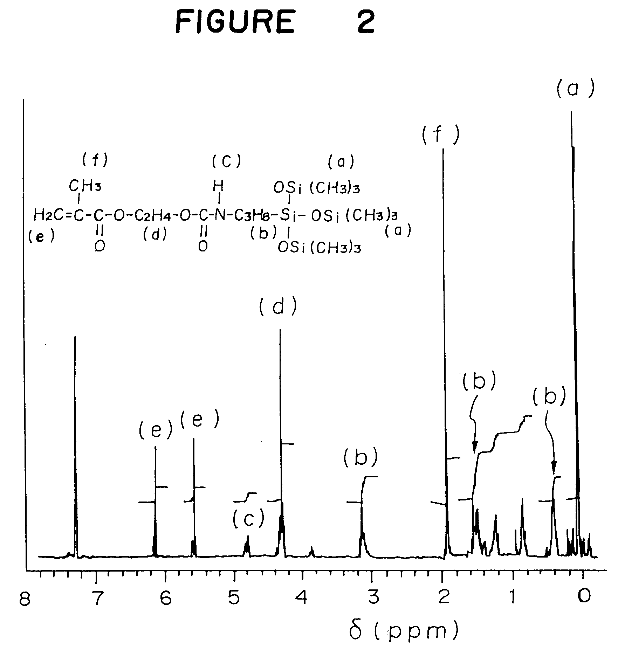

Further, with respect to the obtained product, the ¹H-NMR spectrum was measured by means of super conductive FR-NMR GEMINI 2000/400 manufactured by Varian Company. The results are shown in Figure 2.

The results shown in Figure 2 indicate that the signal (peak (a) in Figure 2) attributable to -Si(CH₃) was observed in the vicinity of 0.15 ppm, the signals (peaks (b) in Figure 2) attributable to -C₃H₆- were observed in the vicinity of 0.41 ppm, 1.58 ppm and 3.15 ppm, the signal (peak (c) in Figure 2) attributable to -NH- was observed in the vicinity of 4.81 ppm, the signal (peak (d) in Figure 2) attributable to -C₂H₄- was observed in the vicinity of 4.30 ppm, the signals (peaks (e) in Figure 2) attributable to =CH₂ were observed in the vicinity of 5.95 ppm and 6.18 ppm, and the signal (peak (f) in Figure 2) attributable to -C(CH₃)= was observed in the vicinity of 1.98 ppm.

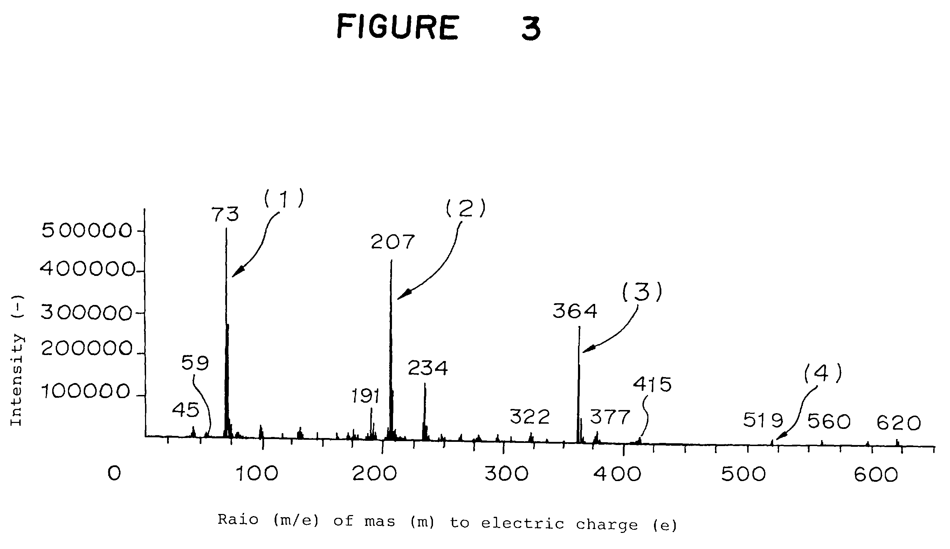

Furthermore, with respect to the obtained product, the mass spectrum was measured by means of GC-MS (5890 II Gas Chromatograph) manufactured by Hewlett Packard. The results are shown in Figure 3.

Attribution of peaks (1) to (4) shown in Figure 3 is as follows:
Peak (1): 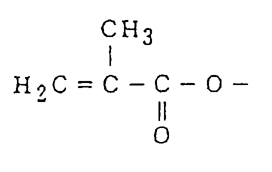
Peak (2): 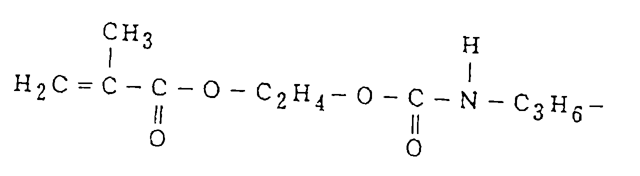
Peak (3): 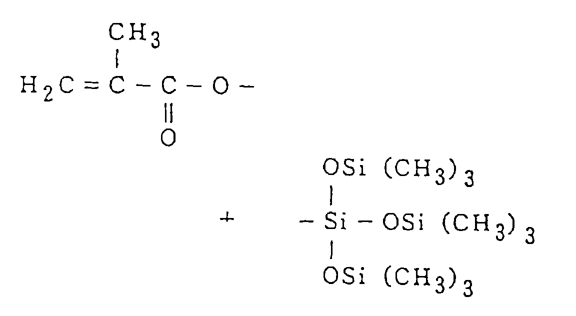
Peak (4):

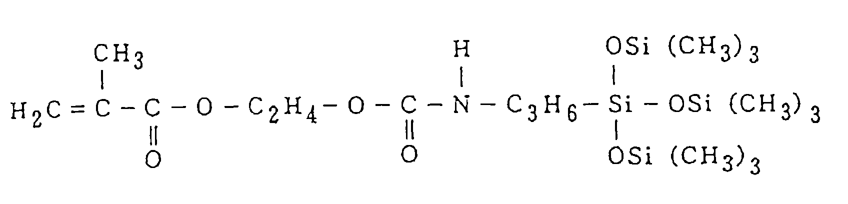

From these results, the obtained product was ascertained to be N-tris(trimethylsiloxy)silylpropylmethacryloxyethyl carbamate of the formula:

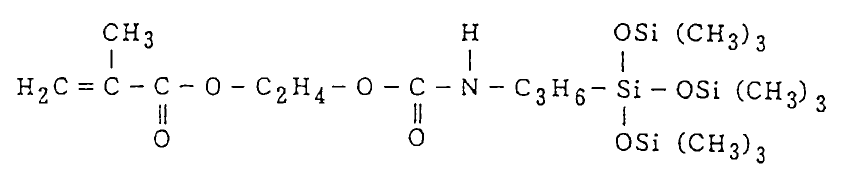

### EXAMPLE 2

### Synthesis of N-tris(trimethylsiloxy)silylpropylacryloxyethyl carbamate

70 g (yield: 83%) of a colorless transparent liquid product was prepared in the same manner as in Example 1 except that in Example 1, instead of 50 g (about 0.4 mol) of 2-hydroxyethyl methacrylate, 50 g (about 0.4 mol) of 2-hydroxyethyl acrylate was used.

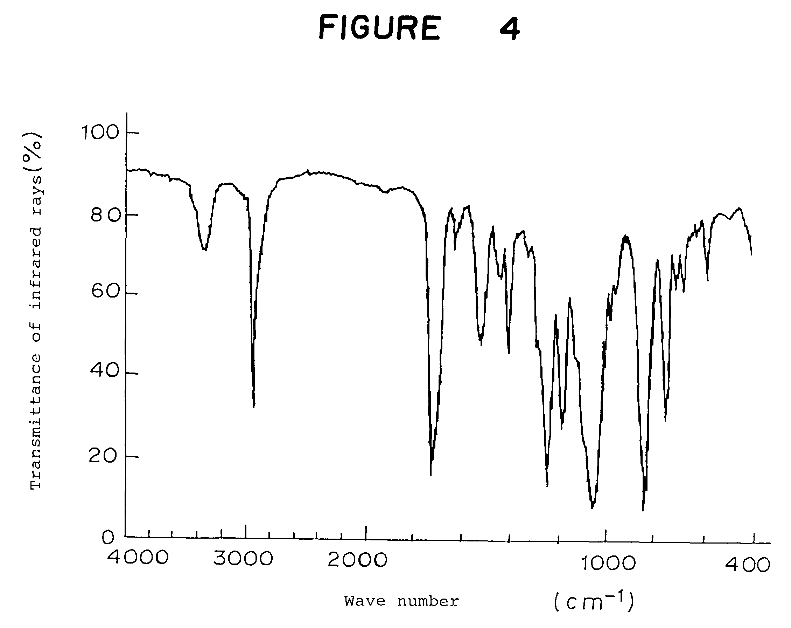

With respect to the obtained product, the infrared absorption spectrum was measured in the same manner as in Example 1. The results are shown in Figure 4 as the transmittance (%) of infrared rays.

The results shown in Figure 4 indicate that the absorption attributable to a urethane bond (-OCONH-) was observed in the vicinity of 3,400 cm⁻¹ and 1,750 cm⁻¹, the absorption attributable to Si-O-Si was observed in the vicinity of 1,054 cm⁻¹, and the absorption attributable to (CH₃)₃-SiO- was observed in the vicinity of 843 cm⁻¹.

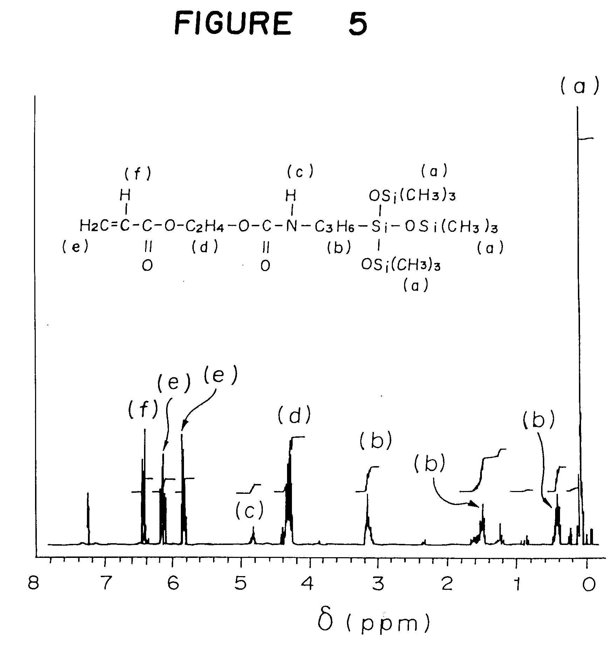

Further, with respect to the obtained product, the ¹H-NMR spectrum was measured in the same manner as in Example 1. The results are shown in Figure 5.

The results shown in Figure 5 indicate that the signal (peak (a) in Figure 5) attributable to -Si(CH₃)₃ was observed in the vicinity of 0.15 ppm, the signals (peaks (b) in Figure 5) attributable to -C₃H₆- were observed in the vicinity of 0.41 ppm, 1.58 ppm and 3.15 ppm, the signal (peak (c) in Figure 5) attributable to -NH- was observed in the vicinity of 4.81 ppm, the signal (peak (d) in Figure 5) attributable to -C₂H₄- was observed in the vicinity of 4.30 ppm, the signals (peaks (e) in Figure 5) attributable to =CH₂ was observed in the vicinity of 5.83 ppm and 6.18 ppm, and the signal (peak (f) in Figure 5) attributable to -CH= was observed in the vicinity of 6.41 ppm.

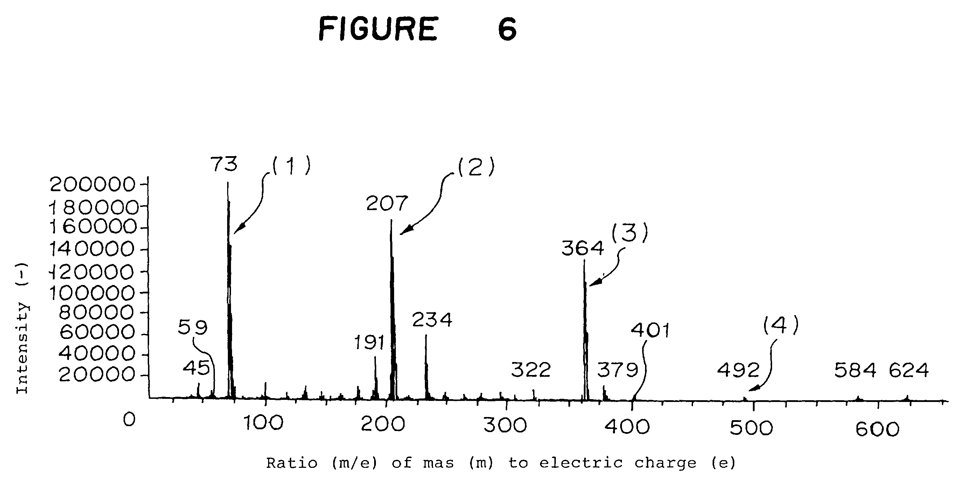

Furthermore, with respect to the obtained product, the mass spectrum was measured in the same manner as in Example 1. The results are shown in Figure 6.

Attribution of peaks (1) to (4) shown in Figure 6 is as follows:
Peak (1): 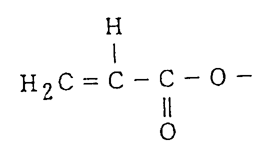
Peak (2): 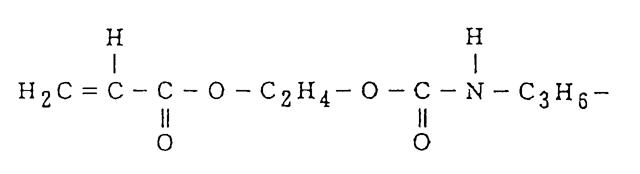
Peak (3): 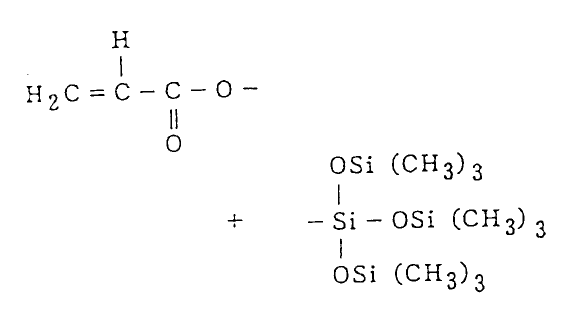
Peak (4):

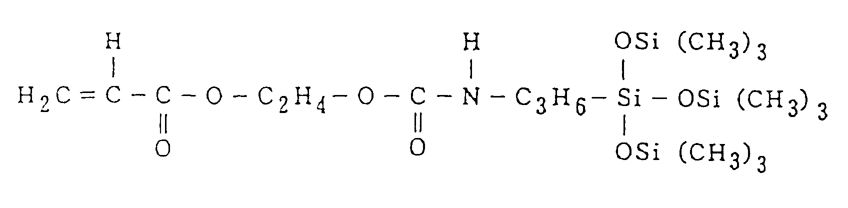

From these results, the obtained product was ascertained to be N-tris(trimethylsiloxy)silylpropylacryloxyethyl carbamate of the formula:

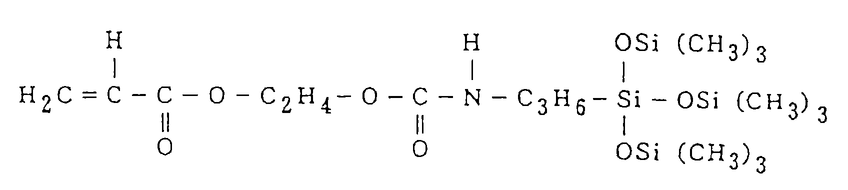

### EXAMPLE 3

### Preparation of a polymer

As polymerizable components, 100 parts by weight of N-tris(trimethylsiloxy)silylpropylmethacryloxyethyl carbamate obtained in Example 1 and 0.3 part by weight of ethylene glycol dimethacrylate, and as a polymerization initiator, 0.1 part by weight of 2,2'-azobis(2,4-dimethylvaleronitrile), were mixed, and the obtained mixture was transferred to a test tube. The inner atmosphere of the reaction system was substituted by nitrogen, and the test tube was then sealed.

Then, the sealed test tube was immersed in a circulation system constant temperature water tank, whereupon polymerization was carried out at 35°C for 40 hours and at 50°C for 8 hours. The test tube was transferred to a circulation system drier and heated from 60 to 120°C for 14 hours while raising the temperature at a rate of 10°C per 2 hours and at 110°C for 2 hours, to complete the polymerization to obtain a colorless transparent polymer.

The obtained polymer was subjected to mechanical processing by cutting, grinding and polishing to obtain a test specimen having a diameter of 15 mm and a thickness of 0.2 mm. The processability of the polymer at that time was excellent.

The obtained test specimen was visually inspected, whereby it was transparent without fogging and free from optical strain, and it was suitable for use, for example, as an ocular lens material.

Further, the above test specimen was subjected to cutting, grinding and polishing to obtain a contact lens. The cut surface and the polished surface of such a contact lens were excellent.

Then, the oxygen permeability coefficient and the contact angle of the above test specimen were measured in accordance with the following methods. The results are shown in Table 1.

### (a) Oxygen permeation coefficient (DK_{0.2})

Using a Seikaken Model film oxygen permeation meter manufactured by Rikaseiki Kogyo K.K., the oxygen permeation coefficient of the test specimen was measured in a physiological sodium chloride aqueous solution at 35°C. The unit of the oxygen permeation coefficient is ml (STP)·cm²/(cm³·sec·mmHg), and the oxygen permeation coefficient in Table 1 is a numerical value obtained by multiplying 10¹¹ by the value of the oxygen permeation coefficient when the thickness of the test specimen is 0.2 mm.

### (b) Contact angle

Contact angle (degree) was measured by a liquid drop method at a temperature of 25°C by means of a goniometer.

### EXAMPLES 4 TO 13 AND COMPARATIVE EXAMPLE 1

A colorless transparent polymer was prepared in the same manner as in Example 3 except that in Example 3, the polymerizable components were changed as shown in Table 1.

From the obtained polymer, a test specimen was prepared in the same manner as in Example 3. The processability of the polymer in each of Examples 4 to 13 was excellent.

The obtained test specimen was visually inspected. The test specimens of Examples 4 to 13 were transparent without fogging and free from optical strain, and they were suitable for use, for example, as ocular lens materials.

Further, the test specimens were subjected to cutting, grinding and polishing to obtain contact lenses. The cutting surface and the polishing surface of each of the contact lenses of Examples 4 to 13 were excellent.

Then, the oxygen permeation coefficients and the contact angles of the test specimens were measured in the same manner as in Example 3. The results are shown in Table 1.

The abbreviations in Table 1 represent the following compounds.
- TTMSSPMAEC:: N-tris(trimethylsiloxy)silylpropyl methacryloxyethyl carbamate (compound prepared in Example 1)
- TTMSSPAEC:: N-tris(trimethylsiloxy)silylpropyl methacryloxyethyl carbamate (compound prepared in Example 2)
- TTMSSPMA:: Tris(trimethylsiloxy)silylpropyl methacrylate
- DMAA:: N,N-dimethylacrylamide
- MMA:: Methyl methacrylate
- BuA:: n-Butyl acrylate
- EDMA:: Ethylene glycol dimethacrylate

**Table 1**

| Example No. | Polymerizable components | | | | | | | Physical properties of test specimens | |
|---|---|---|---|---|---|---|---|---|---|
| | TTMSSPMAEC | TTMSSPAEC | TTMSSPMA | DMAA | MMA | BuA | EDMA | Oxygen permeation coefficient (DK⁰²) | Contact angle (degree) |
| 1 | 100 | - | - | - | - | - | 0.3 | 62 | 60 |
| 2 | 75 | - | - | 25 | - | - | 0.3 | 47 | 45 |
| 3 | 50 | - | - | 50 | - | - | 0.3 | 35 | 35 |
| 4 | 25 | - | - | 75 | - | - | 0.3 | 45 | 25 |
| 5 | 75 | - | - | - | 25 | - | 0.3 | 68 | 60 |
| 6 | 50 | - | - | - | 50 | - | 0.3 | 64 | 63 |
| 7 | 25 | - | - | - | 75 | - | 0.3 | 24 | 65 |
| 8 | 75 | - | - | - | - | 25 | 0.3 | 65 | 60 |
| 9 | 50 | - | - | - | - | 50 | 0.3 | 53 | 63 |
| 10 | 25 | - | - | - | - | 75 | 0.3 | 48 | 65 |
| 11 | - | 100 | - | - | - | - | 0.3 | 65 | 60 |
| Comparative Example 1 | - | - | 100 | - | - | - | 0.3 | 110 | 120 |

It is evident from the results shown in Table 1 that the polymer (the test specimen) of Comparative Example 1 obtained by using a conventional silicon-containing methacrylate only, has an extremely large contact angle of 120° and thus is very poor in wettability (hydrophilic property) although its oxygen permeation coefficient is large. Whereas, the polymers (the test specimens) of Examples 3 to 13 obtained by using the silicon-containing compounds of Examples 1 and 2, have small contact angles and are thus excellent in wettability (hydrophilic properties), while their oxygen permeation coefficients are comparable to the oxygen permeation coefficient of the polymer of Comparative Example 1.

As described in the foregoing, the silicon-containing compound of the present invention is a compound which is excellent in polymerizability and which is capable of simultaneously imparting excellent oxygen permeability, transparency and surface hydrophilic property to the resulting polymer and has a nature of imparting release property, dimensional stability, durability, sliding property, low friction property, anti-thrombogenic property, etc. Accordingly, the polymer obtained by using the silicon-containing compound as a polymerizable component, is useful not only for e.g. a fiber-treating agent, a coating material, a silicon-modified rubber material or a surface-treating agent, but also for a highly oxygen permeable molded product, particularly an ocular lens material such as a contact lens material, an intraocular lens material or an artificial cornea material.

Further, the ocular lens material of the present invention is excellent in oxygen permeability and transparency and simultaneously has a proper hydrophilic property, and at the same time, it is excellent in e.g. release property, dimensional stability, durability, sliding property, low friction property and anti-thrombogenic property. Accordingly, the ocular lens material of the present invention is useful particularly for e.g. a contact lens, an intraocular lens and an artificial cornea.

## Claims

1. A silicon-containing compound of the formula (I): 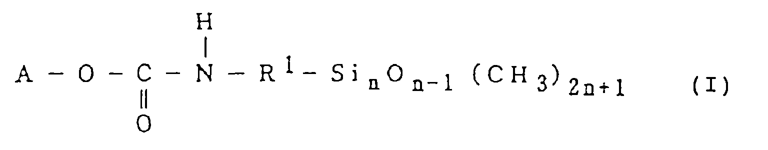 wherein R¹ is a C₁₋₆ alkylene group; A is a group of the formula (II): 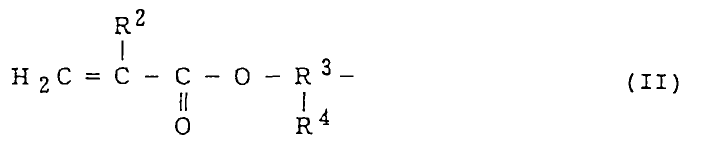 wherein R² is a hydrogen atom or a methyl group, R³ is a C₁₋₆ trivalent hydrocarbon group or a C₁₋₆ bivalent hydrocarbon group when R⁴ is a hydrogen atom, and R⁴ is a hydrogen atom, a hydroxyl group, a C₁₋₆ alkyl group or a group of the formula (III): 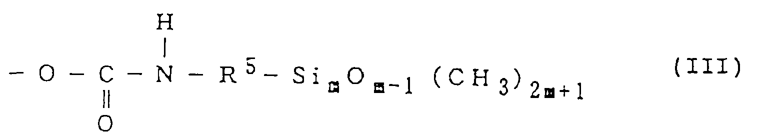 wherein R⁵ is a C₁₋₆ alkylene group, and m is an integer of from 1 to 15, or a group of the formula (IV): 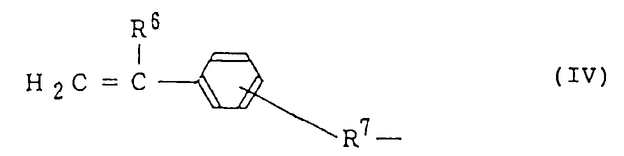 wherein R⁶ is a hydrogen atom or a methyl group, and R⁷ is a direct bond or a C₁₋₆ alkylene group; and n is an integer of from 1 to 15.

2. An ocular lens material made of a polymer obtained by polymerizing polymerizable components containing a silicon-containing compound of the formula (I): 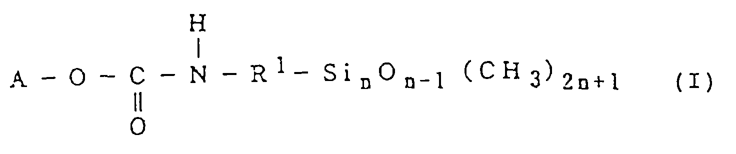 wherein R¹ is a C₁₋₆ alkylene group; A is a group of the formula (II): 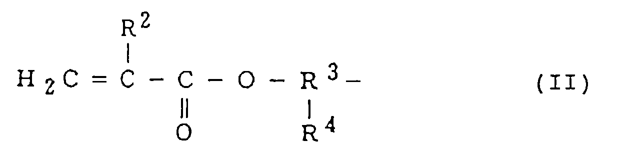 wherein R² is a hydrogen atom or a methyl group, R³ is a C₁₋₆ trivalent hydrocarbon group or a C₁₋₆ bivalent hydrocarbon group when R⁴ is a hydrogen atom, and R⁴ is a hydrogen atom, a hydroxyl group, a C₁₋₆ alkyl group or a group of the formula (III): 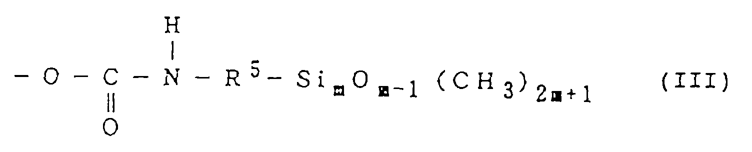 wherein R⁵ is a C₁₋₆ alkylene group, and m is an integer of from 1 to 15, or a group of the formula (IV): 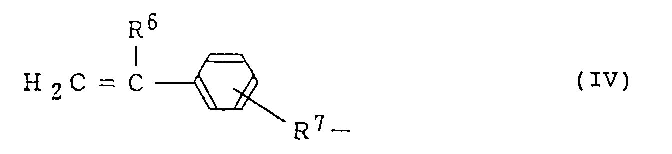 wherein R⁶ is a hydrogen atom or a methyl group, and R⁷ is a direct bond or a C₁₋₆ alkylene group; and n is an integer of from 1 to 15.

3. The ocular lens material according to Claim 2, wherein the content of the silicon-containing compound is at least 5 wt% of the total amount of the polymerizable components.

4. The ocular lens material according to Claim 2 or 3, wherein the polymerizable components contain a polymerizable monomer having an unsaturated double bond copolymerizable with the silicon-containing compound.

5. The ocular lens material according to Claim 4, wherein the polymerizable monomer having an unsaturated double bond copolymerizable with the silicon-containing compound, is a crosslinkable monomer.

6. The ocular lens material according to Claim 5, wherein the content of the crosslinkable monomer is from 0.01 to 10 wt% of the total amount of the polymerizable components.
